# EUROPEAN PATENT SPECIFICATION

(11) **EP 0 506 923 B1**
(45) Date of publication and mention of the grant of the patent: **19.06.1996**
(21) Application number: 91918842.5
(22) Date of filing: 22.10.1991
(51) Int. Cl.: C07K 14/31

(54) **A COLLAGEN BINDING PROTEIN AS WELL AS ITS PREPARATION**
KOLLAGEN-BINDENDES PROTEIN SOWIE DESSEN HERSTELLUNG
PROTEINE DE LIAISON DU COLLAGENE ET PREPARATION DE CETTE PROTEINE

(30) Priority: 22.10.1990 SE 9003374
(43) Date of publication of application: 07.10.1992
(73) Proprietor: ALFA-LAVAL AGRI INTERNATIONAL AB, S-147 00 Tumba (SE)
(72) Inventor: GUSS, Bengt, Mikael, S-756 52 Uppsala (SE); HÖÖK, Magnus, Birmingham, AL 35244 (US); JONSSON, Hans, S-752 29 Uppsala (SE); LINDBERG, Kjell, Martin, S-752 57 Uppsala (SE); PATTI, Joseph, University of Alabama at Birmingham, Birmingham, AL 35294 (US); SIGNÄS, Lars, Christer, S-753 23 Uppsala (SE); SWITALSKI, Lech, M., University of Alabama at, BHSB 508, Birmingham, AL 35294 (US)
(74) Representative: Inger, Lars Ulf Bosson
(86) International application number: SE9100707
(87) International publication number: WO9207002

(56) References cited:
- EP-A- 0 163 623
- Dialog Information Services, file 55: BIOSIS 85-92, Dialog accession No. 7397648, L.M. SWITALSKI et al.: "Isolation and characterization of a putative collagen receptor from staphylococcus-aureus strain cowan 1", & J Biol. Chem 264 (35) 1989 21080-21086.

## Description

### A COLLAGEN BINDING PROTEIN AS WELL AS ITS PREPARATION.

### Technical field

The present invention relates to a collagen binding protein as well as hybrid-DNA-molecules, e.g. plasmids or phages comprising a nucleotide sequence coding for said protein. Further the invention relates to micro-organisms comprising said molecules and their use producing said protein, as well as the synthetic preparation of said protein. In particular the invention relates to a cloned gene encoding the Staphylococcus aureus collagen binding protein, or functionally active portions thereof, vectors containing the cloned gene or parts thereof, and micro-organisms transformed by those vectors as well as the cloning of the gene which specify the biosynthesis of Staphylococcus aureus collagen binding protein (CBP) (also called the collagen receptor by Switalski et al 1989) and the use of organisms transformed with the cloned gene to produce CBP or CBP like proteins. The invention also describes the use of this gene for diagnostic purposes.

The object of the present invention is to obtain a collagen binding protein.

A further object is to obtain said protein by means of a genetic engineering technique by using e.g. a plasmid comprising a nucleotide sequence coding for said protein.

A further object is to obtain a possibility of preparing said protein by chemical synthesis.

Further objects will be apparent from the following description.

### Background of the invention

WO-A1-85/05553 discloses bacterial cell surface proteins having fibronectin, fibrinogen, collagen, and/or laminin binding ability. Thereby it is shown that different bacteria have an ability to bind to fibronectin, fibrinogen, collagen, and/or laminin.

Regarding the binding of collagen to S. aureus several studies have been reported (Carret et al 1985, Holderbaum et al 1985, Holderbaum et al 1986, Vercellotti et al 1985, Speziale et al 1986, Switalski et al 1989).

Switalski et al 1989 reported on the isolation and characterization of a S. aureus surface protein which they identified as a collagen receptor. Using lysostaphin to release the protein from the cell wall followed by ion exchange chromatography, ammonium sulfate precipitation and gel filtration it was possible to purify a protein with an apparent Mr of 135 kDa. It was also shown that antibodies raised against the 135 kDa protein inhibited the binding of collagen to S. aureus Cowan 1 cells.

### Description of the invention

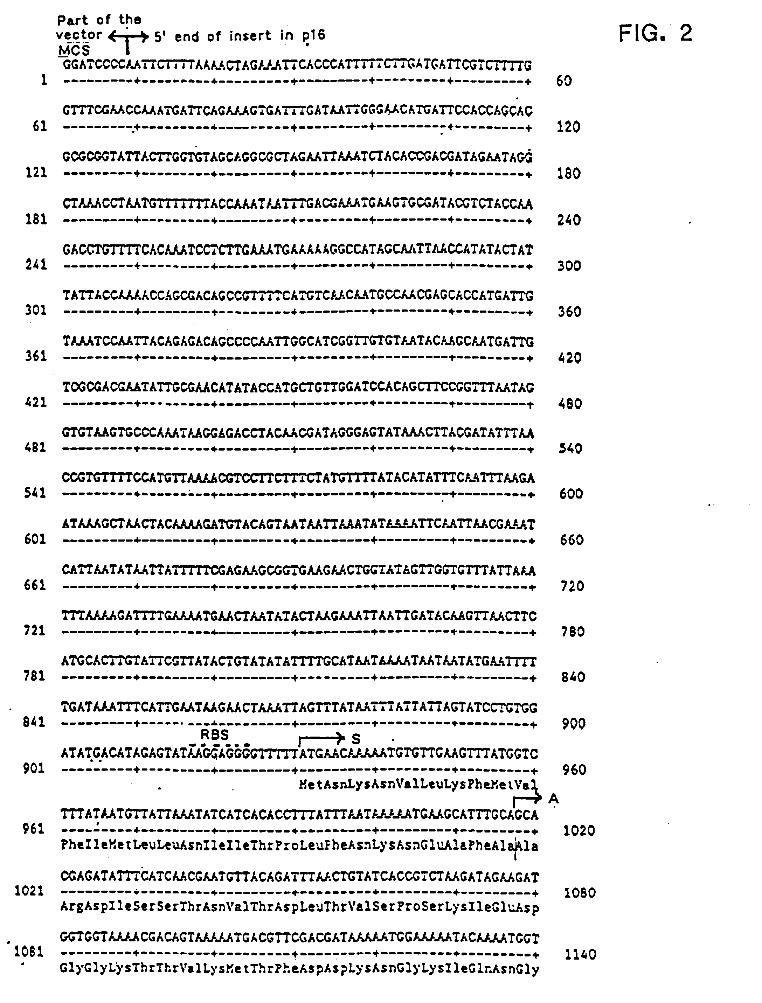

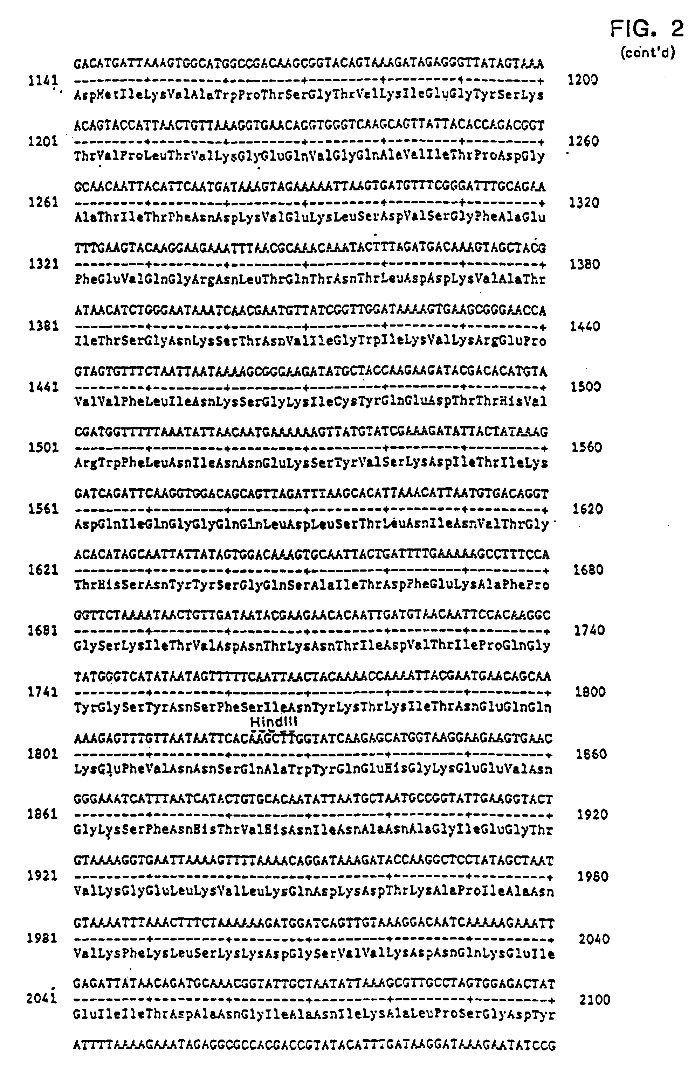

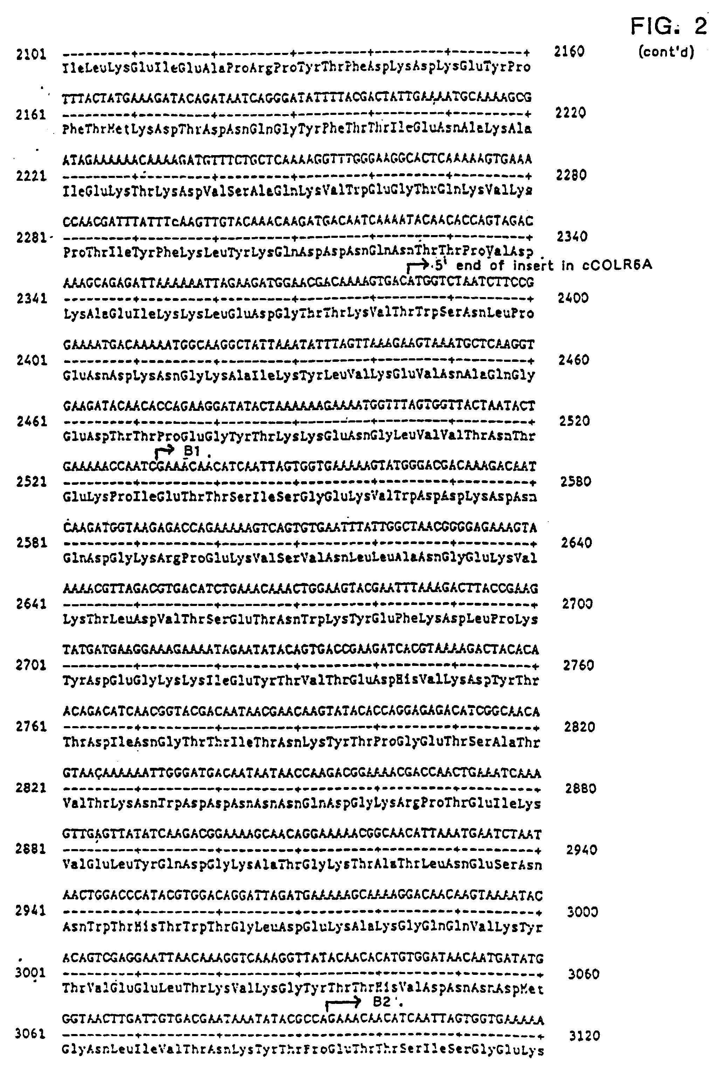

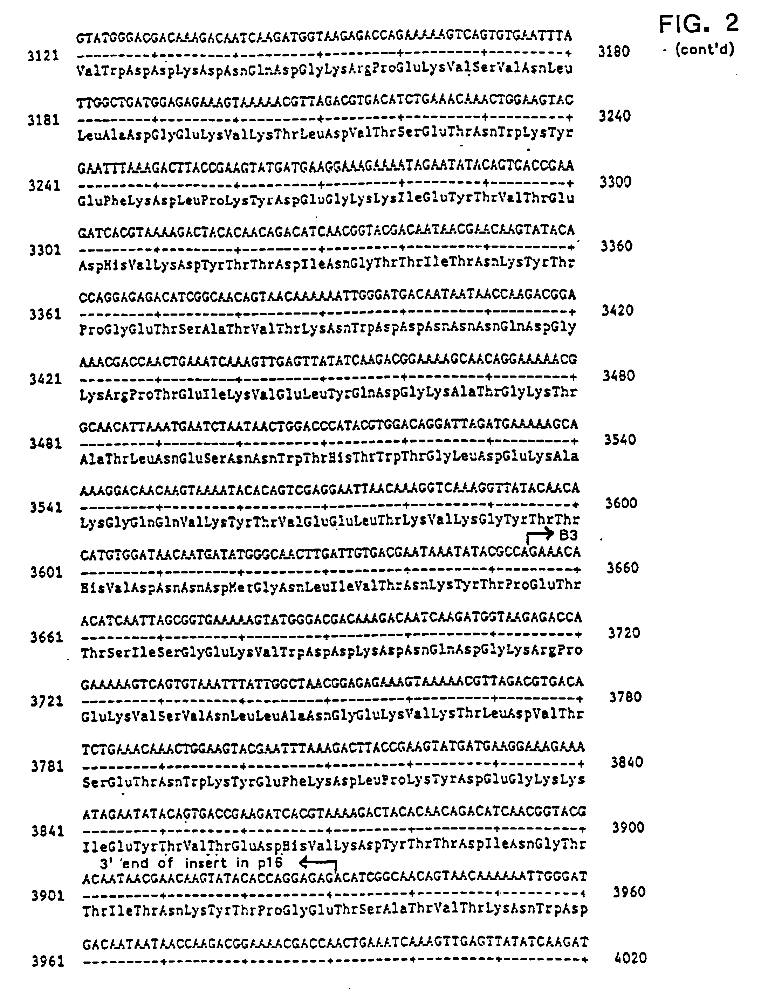

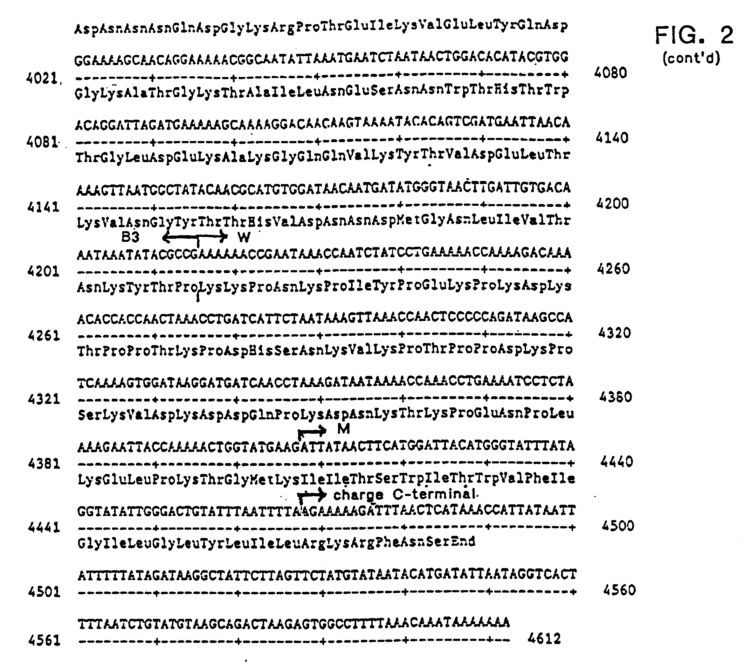

It has now surprisingly been found possible to obtain a hybride-DNA-molecule comprising a nucleotide sequence coding for a protein or a polypeptide having collagen binding properties. As evident from below the following nucleotide sequence is present in the gene coding for said protein. 
GCA
CGAGATATTT CATCAACGAA TGTTACAGAT TTAACTGTAT CACCGTCTAA
GATAGAAGAT GGTGGTAAAA CGACAGTAAA AATGACGTTC GACGATAAAA
ATGGAAAAAT ACAAAATGGT GACATGATTA AAGTGGCATG GCCGACAAGC
GGTACAGTAA AGATAGAGGG TTATAGTAAA ACAGTACCAT TAACTGTTAA
AGGTGAACAG GTGGGTCAAG CAGTTATTAC ACCAGACGGT GCAACAATTA
CATTCAATGA TAAAGTAGAA AAATTAAGTG ATGTTTCGGG ATTTGCAGAA
TTTGAAGTAC AAGGAAGAAA TTTAACGCAA ACAAATACTT TAGATGACAA
AGTAGCTACG ATAACATCTG GGAATAAATC AACGAATGTT ATCGGTTGGA
TAAAAGTGAA GCGGGAACCA GTAGTGTTTC TAATTAATAA AAGCGGGAAG
ATATGCTACC AAGAAGATAC GACACATGTA CGATGGTTTT TAAATATTAA
CAATGAAAAA AGTTATGTAT CGAAAGATAT TACTATAAAG GATCAGATTC
AAGGTGGACA GCAGTTAGAT TTAAGCACAT TAAACATTAA TGTGACAGGT
ACACATAGCA ATTATTATAG TGGACAAAGT GCAATTACTG ATTTTGAAAA
AGCCTTTCCA GGTTCTAAAA TAACTGTTGA TAATACGAAG AACACAATTG
ATGTAACAAT TCCACAAGGC TATGGGTCAT ATAATAGTTT TTCAATTAAC
 
TACAAAACCA AAATTACGAA TGAACAGCAA AAAGAGTTTG TTAATAATTC
ACAAGCTTGG TATCAAGAGC ATGGTAAGGA AGAAGTGAAC GGGAAATCAT
TTAATCATAC TGTGCACAAT ATTAATGCTA ATGCCGGTAT TGAAGGTACT
GTAAAAGGTG AATTAAAAGT TTTAAAACAG GATAAAGATA CCAAGGCTCC
TATAGCTAAT GTAAAATTTA AACTTTCTAA AAAAGATGGA TCAGTTGTAA
AGGACAATCA AAAAGAAATT GAGATTATAA CAGATGCAAA CGGTATTGCT
AATATTAAAG CGTTGCCTAG TGGAGACTAT ATTTTAAAAG AAATAGAGGC
GCCACGACCG TATACATTTG ATAAGGATAA AGAATATCCG TTTACTATGA
AAGATACAGA TAATCAGGGA TATTTTACGA CTATTGAAAA TGCAAAAGCG
ATAGAAAAAA CAAAAGATGT TTCTGCTCAA AAGGTTTGGG AAGGCACTCA
AAAAGTGAAA CCAACGATTT ATTTCAAGTT GTACAAACAA GATGACAATC
AAAATACAAC ACCAGTAGAC AAAGCAGAGA TTAAAAAATT AGAAGATGGA
ACGACAAAAG TGACATGGTC TAATCTTCCG GAAAATGACA AAAATGGCAA
GGCTATTAAA TATTTAGTTA AAGAAGTAAA TGCTCAAGGT GAAGATACAA
CACCAGAAGG ATATACTAAA AAAGAAAATG GTTTAGTGGT TACTAATACT
GAAAAACCAA TCGAAACAAC ATCAATTAGT GGTGAAAAAG TATGGGACGA
CAAAGACAAT CAAGATGGTA AGAGACCAGA AAAAGTCAGT GTGAATTTAT
TGGCTAACGG GGAGAAAGTA AAAACGTTAG ACGTGACATC TGAAACAAAC
TGGAAGTACG AATTTAAAGA CTTACCGAAG TATGATGAAG GAAAGAAAAT
AGAATATACA GTGACCGAAG ATCACGTAAA AGACTACACA ACAGACATCA
ACGGTACGAC AATAACGAAC AAGTATACAC CAGGAGAGAC ATCGGCAACA
GTAACAAAAA ATTGGGATGA CAATAATAAC CAAGACGGAA AACGACCAAC
TGAAATCAAA GTTGAGTTAT ATCAAGACGG AAAAGCAACA GGAAAAACGG
CAACATTAAA TGAATCTAAT AACTGGACCC ATACGTGGAC AGGATTAGAT
GAAAAAGCAA AAGGACAACA AGTAAAATAC ACAGTCGAGG AATTAACAAA
GGTCAAAGGT TATACAACAC ATGTGGATAA CAATGATATG GGTAACTTGA
TTGTGACGAA TAAATATACG CCAGAAACAA CATCAATTAG TGGTGAAAAA
GTATGGGACG ACAAAGACAA TCAAGATGGT AAGAGACCAG AAAAAGTCAG
TGTGAATTTA TTGGCTGATG GAGAGAAAGT AAAAACGTTA GACGTGACAT
CTGAAACAAA CTGGAAGTAC GAATTTAAAG ACTTACCGAA GTATGATGAA
GGAAAGAAAA TAGAATATAC AGTGACCGAA GATCACGTAA AAGACTACAC
AACAGACATC AACGGTACGA CAATAACGAA CAAGTATACA CCAGGAGAGA
CATCGGCAAC AGTAACAAAA AATTGGGATG ACAATAATAA CCAAGACGGA
AAACGACCAA CTGAAATCAA AGTTGAGTTA TATCAAGACG GAAAAGCAAC
AGGAAAAACG GCAACATTAA ATGAATCTAA TAACTGGACC CATACGTGGA
CAGGATTAGA TGAAAAAGCA AAAGGACAAC AAGTAAAATA CACAGTCGAG
 
GAATTAACAA AGGTCAAAGG TTATACAACA CATGTGGATA ACAATGATAT
GGGCAACTTG ATTGTGACGA ATAAATATAC GCCAGAAACA ACATCAATTA
GCGGTGAAAA AGTATGGGAC GACAAAGACA ATCAAGATGG TAAGAGACCA
GAAAAAGTCA GTGTAAATTT ATTGGCTAAC GGAGAGAAAG TAAAAACGTT
AGACGTGACA TCTGAAACAA ACTGGAAGTA CGAATTTAAA GACTTACCGA
AGTATGATGA AGGAAAGAAA ATAGAATATA CAGTGACCGA AGATCACGTA
AAAGACTACA CAACAGACAT CAACGGTACG ACAATAACGA ACAAGTATAC
ACCAGGAGAG ACATCGGCAA CAGTAACAAA AAATTGGGAT GACAATAATA
ACCAAGACGG AAAACGACCA ACTGAAATCA AAGTTGAGTT ATATCAAGAT
GGAAAAGCAA CAGGAAAAAC GGCAATATTA AATGAATCTA ATAACTGGAC
ACATACGTGG ACAGGATTAG ATGAAAAAGC AAAAGGACAA CAAGTAAAAT
ACACAGTCGA TGAATTAACA AAAGTTAATG GCTATACAAC GCATGTGGAT
AACAATGATA TGGGTAACTT GATTGTGACA AATAAATATA CGCCGAAAAA
ACCGAATAAA CCAATCTATC CTGAAAAACC AAAAGACAAA ACACCACCAA
CTAAACCTGA TCATTCTAAT AAAGTTAAAC CAACTCCCCC AGATAAGCCA
TCAAAAGTGG ATAAGGATGA TCAACCTAAA GATAATAAAA CCAAACCTGA
AAATCCTCTA AAAGAATTAC CAAAAACTGG TATGAAGATT ATAACTTCAT
GGATTACATG GGTATTTATA GGTATATTGG GACTGTATTT AATTTTAAGA
AAAAGATTTA ACTCA
 whereby this nucleotide sequence encodes for the following protein starting at nucleotide no. 1 in the reading above, whereby the prepresent nucleotides shown in Fig. 2 are part of the signal system:

Ala
ArgAspIleSerSerThrAsnValThrAspLeuThrValSerProSerLysIleGluAsp
GlyGlyLysThrThrValLysMetThrPheAspAspLysAsnGlyLysIleGlnAsnGly
AspMetIleLysValAlaTrpProThrSerGlyThrValLysIleGluGlyTyrSerLys
ThrValProLeuThrValLysGlyGluGlnValGlyGlnAlaValIleThrProAspGly
AlaThrIleThrPheAsnAspLysValGluLysLeuSerAspValSerGlyPheAlaGlu
PheGluValGlnGlyArgAsnLeuThrGlnThrAsnThrLeuAspAspLysValAlaThr
IleThrSerGlyAsnLysSerThrAsnValIleGlyTrpIleLysValLysArgGluPro
ValValPheLeuIleAsnLysSerGlyLysIleCysTyrGlnGluAspThrThrHisVal
ArgTrpPheLeuAsnIleAsnAsnGluLysSerTyrValSerLysAspIleThrIleLys
AspGlnIleGlnGlyGlyGlnGlnLeuAspLeuSerThrLeuAsnIleAsnValThrGly
ThrHisSerAsnTyrTyrSerGlyGlnSerAlaIleThrAspPheGluLysAlaPhePro
GlySerLysIleThrValAspAsnThrLysAsnThrIleAspValThrIleProGlnGly

TyrGlySerTyrAsnSerPheSerIleAsnTyrLysThrLysIleThrAsnGluGlnGln
LysGluPheValAsnAsnSerGlnAlaTrpTyrGlnGluHisGlyLysGluGluValAsn
GlyLysSerPheAsnHisThrValHisAsnIleAsnAlaAsnAlaGlyIleGluGlyThr
ValLysGlyGluLeuLysValLeuLysGlnAspLysAspThrLysAlaProIleAlaAsn
ValLysPheLysLeuSerLysLysAspGlySerValValLysAspAsnGlnLysGluIle
GluIleIleThrAspAlaAsnGlyIleAlaAsnIleLysAlaLeuProSerGlyAspTyr
IleLeuLysGluIleGluAlaProArgProTyrThrPheAspLysAspLysGluTyrPro
PheThrMetLysAspThrAspAsnGlnGlyTyrPheThrThrIleGluAsnAlaLysAla
IleGluLysThrLysAspValSerAlaGlnLysValTrpGluGlyThrGlnLysValLys
ProThrIleTyrPheLysLeuTyrLysGlnAspAspAsnGlnAsnThrThrProValAsp
LysAlaGluIleLysLysLeuGluAspGlyThrThrLysValThrTrpSerAsnLeuPro
GluAsnAspLysAsnGlyLysAlaIleLysTyrLeuValLysGluValAsnAlaGlnGly
GluAspThrThrProGluGlyTyrThrLysLysGluAsnGlyLeuValValThrAsnThr
GluLysProIleGluThrThrSerIleSerGlyGluLysValTrpAspAspLysAspAsn
GlnAspGlyLysArgProGluLysValSerValAsnLeuLeuAlaAsnGlyGluLysVal
LysThrLeuAspValThrSerGluThrAsnTrpLysTyrGluPheLysAspLeuProLys
TyrAspGluGlyLysLysIleGluTyrThrValThrGluAspHisValLysAspTyrThr
ThrAspIleAsnGlyThrThrIleThrAsnLysTyrThrProGlyGluThrSerAlaThr
ValThrLysAsnTrpAspAspAsnAsnAsnGlnAspGlyLysArgProThrGluIleLys
ValGluLeuTyrGlnAspGlyLysAlaThrGlyLysThrAlaThrLeuAsnGluSerAsn
AsnTrpThrHisThrTrpThrGlyLeuAspGluLysAlaLysGlyGlnGlnValLysTyr
ThrValGluGluLeuThrLysValLysGlyTyrThrThrHisValAspAsnAsnAspMet
GlyAsnLeuIleValThrAsnLysTyrThrProGluThrThrSerIleSerGlyGluLys
ValTrpAspAspLysAspAsnGlnAspGlyLysArgProGluLysValSerValAsnLeu
LeuAlaAspGlyGluLysValLysThrLeuAspValThrSerGluThrAsnTrpLysTyr
GluPheLysAspLeuProLysTyrAspGluGlyLysLysIleGluTyrThrValThrGlu
AspHisValLysAspTyrThrThrAspIleAsnGlyThrThrIleThrAsnLysTyrThr
ProGlyGluThrSerAlaThrValThrLysAsnTrpAspAspAsnAsnAsnGlnAspGly
LysArgProThrGluIleLysValGluLeuTyrGlnAspGlyLysAlaThrGlyLysThr
AlaThrLeuAsnGluSerAsnAsnTrpThrHisThrTrpThrGlyLeuAspGluLysAla
LysGlyGlnGlnValLysTyrThrValGluGluLeuThrLysValLysGlyTyrThrThr
HisValAspAsnA[?]nAspMetGlyAsnLeuIleValThrAsnLysTyrThrProGluThr
Thr[?]erI[?]eSerGlyGluLysValTrpAspAspLysAs[?]AsnGlnAspGlyLysArgPro
Glu[?]ysValSerValAsnLeuLeuAlaAsnGlyGluLysValLysThrLeuAspValThr
SerGluThrAsnTrpLysTyrGluPheLysAspLeuProLysTyrAspGluGlyLysLys
IleGluTyrThrValThrGluAspHisValLysAspTyrThrThrAspIleAsnGlyThr

ThrIleThrAsnLysTyrThrProGlyGluThrSerAlaThrValThrLysAsnTrpAsp
AspAsnAsnAsnGlnAspGlyLysArgProThrGluIleLysValGluLeuTyrGlnAsp
GlyLysAlaThrGlyLysThrAlaIleLeuAsnGluSerAsnAsnTrpThrHisThrTrp
ThrGlyLeuAspGluLysAlaLysGlyGlnGlnValLysTyrThrValAspGluLeuThr
LysValAsnGlyTyrThrThrHisValAspAsnAsnAspMetGlyAsnLeuIleValThr
AsnLysTyrThrProLysLysProAsnLysProIleTyrProGluLysProLysAspLys
ThrProProThrLysProAspHisSerAsnLysValLysProThrProProAspLysPro
SerLysValAspLysAspAspGlnProLysAspAsnLysThrLysProGluAsnProLeu
LysGluLeuProLysThrGlyMetLysIleIleThrSerTrpIleThrTrpValPheIle
GlyIleLeuGlyLeuTyrLeuIleLeuArgLysArgPheAsnSer

In the single letter amino acid sequence above the following abbreviations have been used
- A: Ala, Alanine
- R: Arg, Arginine
- N: Asn, Asparagine
- D: Asp, Aspartic acid
- C: Cys, Cysteine
- C: Cys, Cystine
- G: Gly, Glycine
- E: Glu, Glutamic acid
- Q: Gln, Glutamine
- H: His, Histidine
- I: Ile, Isoleucine
- L: Leu, Leucine
- K: Lys, Lysine
- M: Met, Methionine
- F: Phe, Phenylalanine
- P: Pro, Proline
- S: Ser, Serine
- T: Thr, Threonine
- W: Trp, Tryptophan
- Y: Tyr, Tyrosine
- V: Val, Valine

The invention further comprises a plasmid or phage comprising a nucleotide sequence coding for said collagen binding protein.

The invention further comprises a microorganism containing at least one hybrid-DNA-molecule according to the above. The plasmid pSAC104 in an E. coli strain TG1 has been deposited at the Deutsche Sammlung von Mikroorganismen (DSM), and has thereby been allocated the deposition number DSM 6199. The present invention provides a cloned gene encoding the CBP having improved CBP-properties as compared with native CBP which is released and purified from S. aureus cells. The gene is derivied from a S. aureus strain and inserted into a cloning vector. Cells of a procaryotic organism which have been transformed with recombinant vectors are disclosed.

The invention further provides the identification of the nucleotide sequence of the gene encoding the CBP here called the cbp-gene. The deduced amino acid sequence reveals a molecule with several distinct features resembling staphylococcal cell surface proteins.

The invention also provides a procedure for production and purification of the recombinant CBP. This is done in a way so that the molecule retains its collagen binding properties, thus this recombinant CBP resemblance the native unreleased S. aureus CBP.

The invention further provides the use of the cbp-gene for diagnostic purposes. Gene probes chosen to specifically recognize the presence of the cbp gene in clinical S. aureus isolates have been used. As an example, the correlation between the presence of CBP on the surface of S. aureus strains isolated from patient with septic arthritis could be verified by the presence of the cbp-gene in all tested strains.

Appropriate carrier proteins can be coupled to the amino acid sequence as well, such as IgG binding regions of protein A.

The invention will be described in the following with reference to the examples given, however, without being restricted thereto.

### Brief description of the figures

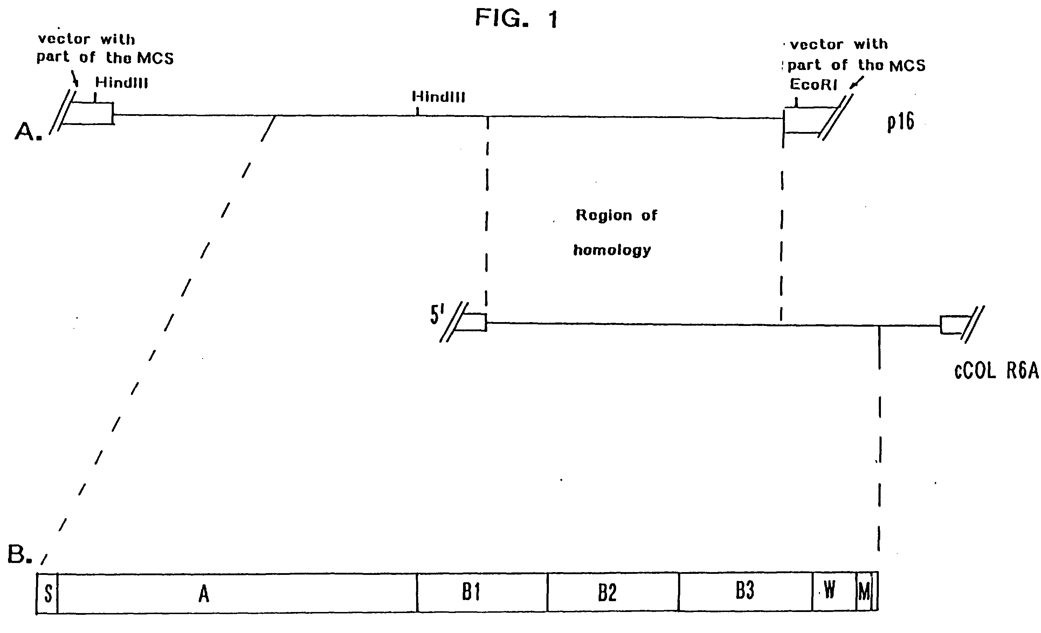

Figure 1: (A), Simplified restriction map of the insert in p 16 and cCOLR6A showing the region of homology, MCS is an abbreviation for multi cloning site. (B), Schematic drawing of the cbp-gene encoding the different regions. S is the proposed signal sequence followed by region A and the repetitive B regions, W is the cell wall spanning region and M the membrane anchoring region.

Figure 2: Nucleotide sequence and the deduced amino acid sequence of the assembled sequence from the insert in p 16 and cCOLR6A. The different regions are marked by arrows and sequences resembling ribosomal binding sites (RBS). The 5'end and 3'end of the insert in p 16 as well as the 5'end of the insert in cCOLR6A is indicated.

### Figure legends

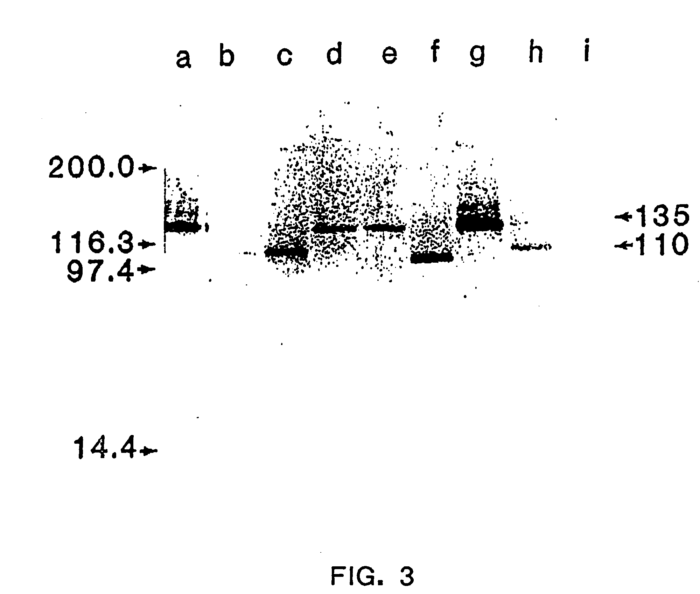

Figure 3: Western blot of lysates of clinical isolates of S. aureus probed with anti-collagen adhesin antibodies. Lysostaphin lysates of strains were separated by gel electrophoresis, electroblotted onto an Immobilon-P membrane. Lanes a: Cowan, b:#7, c:#12, d:#13, e:#14, f: #15, g:#16, h: Phillips, and i:#9.

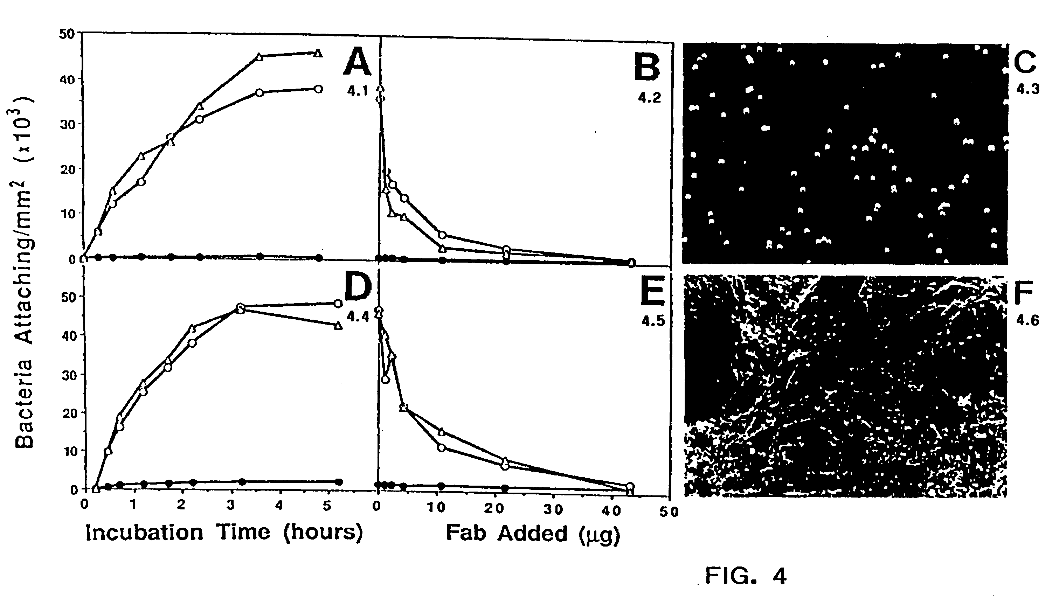

Figure 4: Time dependent attachment of collagen adhesin positive and negative strains of S. aureus to collagen (panel A) and cartilage (panel D). Inhibition of this attachment by ant-adhesin antibodies (panels B and D, collagen and cartilage, respectively). ¹²⁵I-labeled cells of two collagen adhesin positive strains - S. aureus Phillips (Δ) and #14 (○) and one adhesin negative strain - #9 (●) were incubated with collagen coated wells or with pieces of cartilage for indicated periods of time. Electron microscopy of attachment of collagen adhesin positive S. aureus strain Phillips to cartilage (panel C) and inhibition of this attachment by anti-adhesin antibodies (panel F).

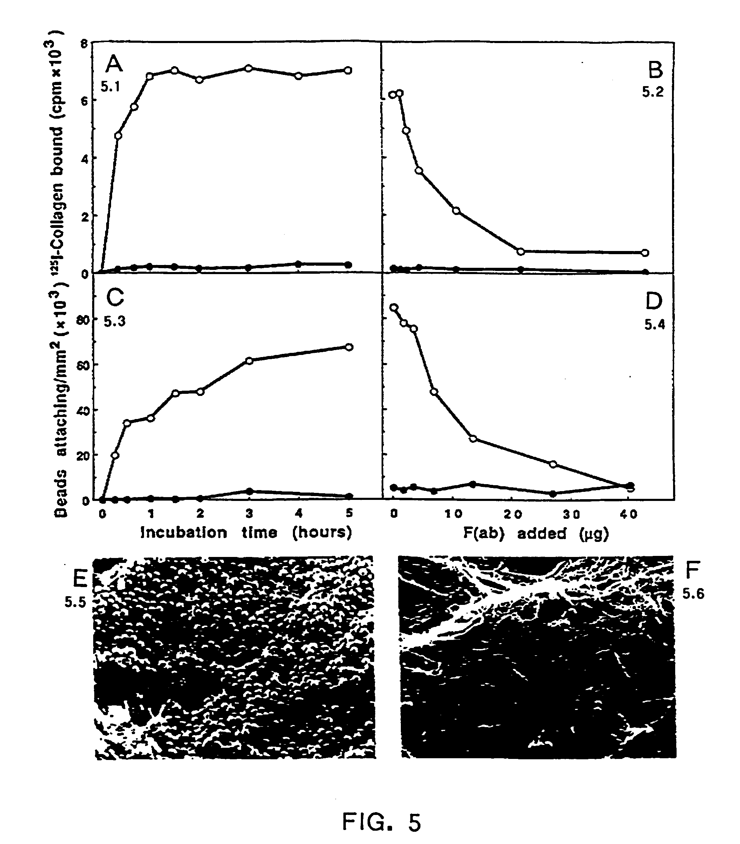

Figure 5: Binding of ¹²⁵I-labeled collagen or adhesion to cartilage by polystyrene beads coated either with the collagen adhesin (○) or a recombinant form of the S. aureus fibronectin receptor (●, ZZFR). Panel A - binding of ¹²⁵I-collagen to protein coated beads as a function of time. Panel B - inhibition of binding of ¹²⁵I-collagen by antibodies. Attachment of ¹²⁵I-labeled beads to cartilage as a function of time (panel C) and inhibition of attachment of ¹²⁵I-labeled beads to cartilage by antibodies (panel D). In this experiment 1 ug of adhesin protein was coupled to 10^{e} polystyrene beads. Control beads were coated with the same molar concentration of the fibronectin receptor. Unreacted sites on the beads were saturated with bovine serum albumin. Scanning electron microscopy of beads coated with collagen adhesin (panel E) or fibronectin receptor protein (panel F) attached to cartilage.

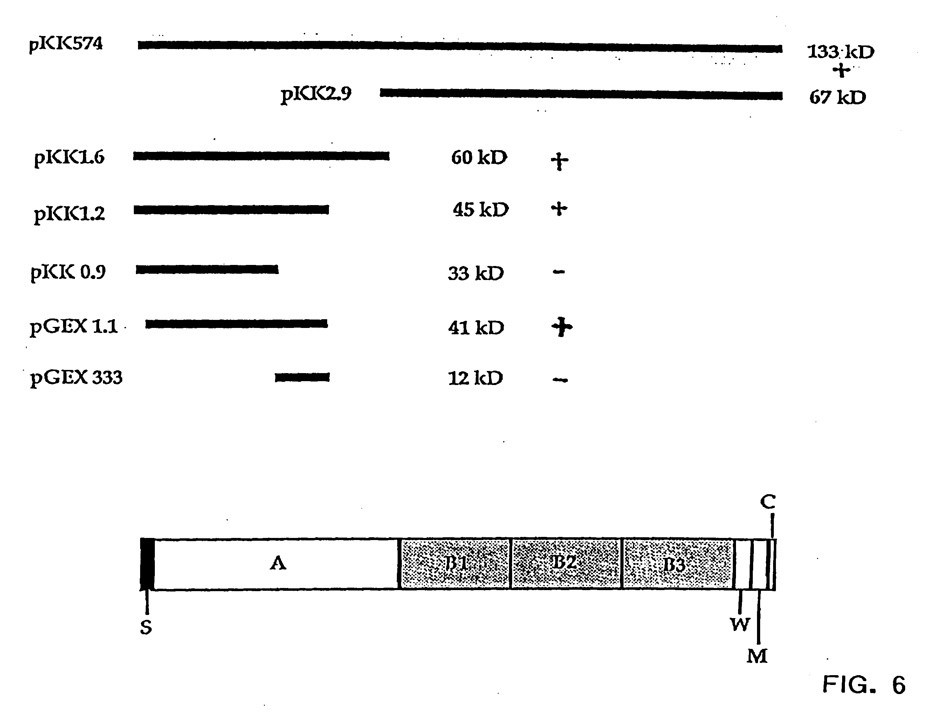

Figure 6: Expression constructs utilized to localize the collagen binding domain within the S. aureus collagen adhesin.

### Example 1:

### Cloning and identification of the cbp-gene in E.coli

In order to isolate the gene encoding S. aureus CBP two commercial available (Clontech laboratories, Inc. Palo Alto, CA, USA) S. aureus strains (strain FDA 574 and FDA 485) were tested if they bound radioactivity labelled collagen. This was done according to Switalski et al 1989. Strain 574 was found to bind collagen and therefore a gene library (obtained from the same company, cat. #XL 15016) of the same strain was screened for the expression of CBP activity. Using the suppliers protocoll (in addition to this protocoll the general work involving molecular genetic appropriate protocolls found in "Current Protocolls in Molecular Biology" Vol. 1 and 2, (edited by Ausubel, F.M., R. Brent. R.E. Kingston, D.D. Moore, I.G. Seidman, J.A. Smith, Struhl, Greene, Wiley Interscience), and "Molecular Cloning". A laboratory manual, (Maniatis, T., Fritsch, E.F. and I. Sambrook (1982) Cold Spring Harbor Laboratory Press, New York) were used) the recombinant lambda gt 11 phages were plated on E. coli strain Y1090. Agarplates with 10.000-100.000 PFU per 90 mm plate was chosen. The plaques from each plate were transferred by replicaplating to nitrocellulose (NC) filters (Schleicher & Schull). To detect plaques expressing CBP activity two different methods were used. In the first method the filters were preincubated in a solution containing 150 mM NaCl; 10 mM Tris pH 7,5; 1,36 % milk powder (defatted) for 1 h at 37 degree C (or overnight at room temperature (RT)). After the incubation the filters were transferred to the same type of solution as above but supplemented with 125-I labelled bovine type II collagen and the filters were incubated over night at RT. The following day the filters were washed 3 X 10 min in a solution containing 150 mM NaCl; 0,05 % Tween 20 at 37 degree C, dried at RT and autoradiographed for several days to detect clones expressing collagen binding activity.

In an alternative screening method purified Fab-fragments from polyclonal rabbit IgG recognizing the native collagen receptor was used to detect clones expressing CBP-activity. This type Fab-fragment preparation had earlier been used by Switalski et al 1989 to identify and characterize the collagen receptor. In this alternative method the replica plated NC-filters were preincubated in a solution containing 150 mM NaCl; 10 mM Tris pH 7,5; 3 % (W/V) bovine serum albumine (BSA) for 45 min at 37 degree to block unspecific binding. After blocking, the filters were transferred to a solution containing phosphate-buffered saline supplemented with Tween 20 to final conc. 0,05 % (PBS-T) which also contained the rabbit anti-collagen receptor Fab-fragments in a dilution of 1:400. After 2,5 h incubation at RT the filters were washed 3 X 10 min in PBS-T followed by the addition of secondary goat anti-rabbit IgG alkaline phosphatase conjugate (Bio-Rad Laboratories, Richmond, CA, USA, Cat. # 170-6518) diluted 1:3000 in PBS-T to detect bound primary Fab-Fragments. After incubation for 1 h at RT the filters were washed 3 X 10 min in PBS-T. The bound labelled secondary antibodies were detected by a color reaction according to the manufacturer's instructions (Bio-Rad, Instructions for preparing the BCIP/NBT color development solution for use in the immun-blot alkaline phosphatase assay kit).

By the use the above described methods several recombinant lambda phages expressing CBP-activity could be identified and isolated.

Two of these were chosen for further studies. They were called lambda coll 1 and lambda cCOLR6A respectively.

Subcloning lambda coll 1: Purified lambda coll 1 DNA was cleaved with EcoRI and the sticky ends were filled in using Klenow fragments together with the dNTP's. The blunt ended DNA-fragments originating from the S. aureus chromosome were ligated into Sma 1 cleaved pUC 18 (Pharmacia-LKB Biotechnology, Uppsala, Sweden). After transformation into freeze competent E. coli TG1 cells recombinant clones were tested for expression of the CBP. It was found that all clones expressing CBP harboured a recombinant plasmid with an insert of approx. 4 kb. One such clone called p 16 was chosen for further studies and a schematic map of the insert in this clone is shown in Fig. 1 A.

In a similar way as lambda coll 1 two other lambda clones were generated from the screening of the genomic library. Large scale cultures of pure positives were obtained and the DNA was isolated. EcoRI digestion of the clones resulted in inserts with two different sizes. Clone 1A had an insert of 3.2 kb and 3B had an insert of 4.5 kb. The larger of the two was used for further characterization. Purified insert DNA (1.5 kb) from λ GT11 clone 3B was ligated to EcoRI digested puc18 and transformed into E. coli TB-1 cell creating subclone cCOLR6A. It was also subcloned into M13mp18/JM101 for sequencing.

CsCl₂ purified plasmid DNA from subclone cCOLR6A was then mapped using a variety restriction endonucleases. Pstl digestion yielded two fragments 2.9 kb and 1.7 kb. Both fragments were sequenced. The 2.9 kb EcoRI-Pstl fragment partially overlaps subclone λ COLL1. Subclone cCOLR6A contains all three repeats, the cell wall domain as well as the membrane spanning domain. A schematic map of the insert in this clone is shown in Fig. 1 A.

Comparative restriction enzyme digestions together with hybridization experiments showed that p 16 and cCOLR6A partially overlapped each other (Fig. 1 A).

### Example 2:

### DNA and amino acid sequence data

In order to determine the nucleotide sequence of the cbp-gene the protocoll included in the Sequenase kit from (United States Biochemical Corporation, USA) was followed in order to analyze the insert in p 16 and cCOOL R6A. By comparing the nucleotide sequence from the inserts it was confirmed that the two inserts were partially homologous (Fig. 1 A and Fig. 2). By assembling these sequences together and searching for open reading frames it was concluded that an open reading frame of 3.555 nt was used corresponding to a deduced amino acid sequence of 1185 amino acids (Fig. 2).

Within the deduced amino acid sequence there are several repetitive and homologous regions. This is schematically shown in Figure 1 B. Starting from the N-terminal end a structure resembling a signal sequence is revealed. This is in agreement with what one should expect since the CBP is a cell surface protein in S. aureus. Following this region, a region called A is found followed by a repetitive strectch of 187 amino acids called B 1, B 2 and B 3. Directly following these regions there is a region called W which consists of a repetitive, hydrophilic structure containing several proline residues. This region resembles a similar structure found in staphylococcal protein A (Guss et al 1984) and FnBP A (Signs et al 1989) as well as streptococcal protein G (Guss et al 1986) and M protein (Hollings-head et al 1986). This region is thought to mediate the binding of the protein to the cell wall. The amino acid sequence nearest to the C-terminal end consists of a long stretch of hydrophobic residues followed by some charged amino acids. This region called M is similar in structure to the C-terminal end of protein A, FnBP A, Protein G and M protein.

The predicted mol.wt of the deduced CBP is approx. 133 kd (including the postulated signal sequence, S) which is very close to the mol.wt of 135 kd reported for the native released receptor (Switalski et al 1989).

In order to construct a plasmid coding for the complete cbp-gene S. aureus FDA 574 chromosomal DNA was purified and double cleaved with Hind III/Pst 1. With the guidance of Southern Transfer experiments using a 32-P labelled oligonucleotide probe (5'-ATTAAAGCGTTGCCTAGTGG-3') it was known that cleavage with these enzymes should generate an approx. 3,2 kb fragment corresponding to the 3'end of the cbp-gene. After cleavage with these enzymes the chromosomal DNA was electophoretically separated in an agarose gel. A gel slice ruffly corresponding to right size was cut out and the DNA fragments eluted and purified. The purified fragments were ligated into pUC 18 previously double cleaved with Hind III/Pst 1. After ligation followed transformation into E. coli TG1 and the resulting recombinant clones were screened for obtaining the right fragment using colony hybridization with the same probe. One positive clone hybridizing with the radioactive probe was chosen for further studies. This clone called E. coli pSAC 100 was cleaved with Hind III and a purified approx. 1,8 kb Hind III fragment from p 16 (encoding the 5'end of the cbp-gene, Fig. 1 A) was ligated into pSAC 100. After transformation into E. coli TG1 recombinant clones having the approx. 1,8 kb fragment in the right orientation was identified and isolated. One such clone called E. coli pSAC 104 was chosen for further studies. The insert in this clone should represent the complete cbp-gene. The clone was also positive when tested for expression of the CBP (see Example 3). This clone is deposited in Deutsche Sammlung von Mikroorganismen, Deposit number 6199.

### Example 3:

### Expression of the CBP in E. coli

Using the 125-I collagen binding assay as described by Switalski et al 1989 E. coli clones containing the whole cbp-gene or parts thereof were tested if lysates from these clones (containing CBP activity) could inhibit 125-I collagen to bind to the S. aureus Cowan I cells. The respective E. coli clone was grown in Luria Broth supplemented with ampicillin final conc. 50 microgram/ml over night. The bacteria were spun down and the supernatant discarded (this since most of the CBP activity was found intracellular). The bacterial pellet was resuspended in 1/10 of the original volume in a solution containing 50 mM Tris pH 8; 50 mM EDTA and lysozyme 1 mg/ml followed by incubation at 37 degree C until complete lysis. The lysed bacteria were centrifuged to remove cellular debris and the supernatant taken care of. The ability of this supernatant (typical volume used was 100-200 microliter) to inhibit 125-I collagen to bind to Cowan I cells was measured. As a control E. coli TG1 pUC 18 treated in the same way was used. The presence of CBP activity could be measured as significant (in some cases up to 66%) reduction in bound radioactive collagen to the Cowan 1 cells when measured in a gamma counter. Three clones measured in this way E. coli TG1 p 16, E. coli TG1 pSAC 104 and E. coli TG1 pCA 1 showed high inhibitory activity as compared with the control E. coli TG1 pUC 18 which showed no significant inhibitory activity. This result is in contrast with the findings reported by Switalski et al 1989 which found that purified or partly purified native collagen receptor could not inhibit the binding of collagen to S. aureus Cowan 1 cells. The conclusion of this is that recombinant CBP expressed has retained more of its original features than the released protein from the staphylococci.

Although it was possible to detect CBP activity in the recombinant E. coli lysate it was not possible to affinity purify the CBP using immobilized collagen or gelatine. Although in "Western transfer" experiments with lysates from the above mentioned recombinant clones, using the Fab-fragments described in Example 1, was it possible to detect bands corresponding to high mol.wt. fragments. These were in the same size as expected from calculations using the deduced amino acid sequence.

### Example 4:

### Expression and of a CBP fusionprotein which retains the collagen binding properties after purification

Been unsuccessful to affinity purify the recombinant produced CBP, using immobilized collagen, another approach was used. This approach was to fuse the cbp-gene or parts of the gene to another gene encoding a so called affinity tail (Methods in enzymology, Part 185). The affinity tail to be tested was the part from the protein A gene encoding the IgG-binding domains (Uhle'n et al 1984). Therefore a vector encoding the above mentioned domains from protein A was used. This vector called pNSEQ1, which was a gift from Dr. M. Uhle'n contains in addition to the IgG-binding domains (E, D, A, B and C) two multi cloning sites (MCS) which flank the IgG-binding domains. This makes it possible to chose a restriction enzyme that has a recognizion site in both the MCS which upon cleavage results in a release of (provided the restriction site is not present in the IgG-binding domains) a DNA fragment encoding the IgG-binding domains which can be purified and inserted into other vectors. Since the nucleotide sequence of the cbp-gene had been determined it was known that p 16 encoded the N-terminal part of the cbp-gene and the decision was to make a C-terminal fusion. This was done in the following way, the p 16 was cleaved with EcoRI (Fig. 1 A) and a purified EcoRI DNA-fragment from pNSEQ1 encoding the IgG-binding part of protein A ligated into the plasmid. After transformation recombinant clones having the right orientation of the inserted protein A fragment were identified and isolated. One of these clones called E. coli pCA 1 was chosen for further studies. It was found that cell lysate of this clone in addition to inhibit collagen binding as measured in Example 3 also showed protein A IgG-binding activity. The next step was to try to affinity purify the presumtive fusionprotein on IgG-Sepharose FF (Pharmacia LKB Biotechnology, Uppsala, Sweden). Using the same manufacturer's Protein A manual it was possible to affinity purify the fusion protein from cell lysate. Using SDS-PAGE to analyse the purified protein it was shown that several bands corresponding to different mol.wt appeared when the gel was stained with Coomassie Brilliant Blue. However, the major band had the corresponding mol.wt of a full length fusionprotein as calculated from the deduced amino acid sequence. When measured for CBP activity this purified protein preparation could inhibit the binding of radioactive collagen to the S. aureus Cowan 1 cells as efficient as the corresponding cell lysate. This is also an improvement as compared with the result presented by Switalski et al 1989. The conclusion is that practizing the presented invention it is now possible to both produce and purify a S. aureus CBP which retains its biological properties in a better way as compared to earlier reported methods.

### Example 5:

### The use of the CBP-gene as a diagnostic tool

Two oligonucleotides (JP-1,5'-AGT-GGT-TAC-TAA-TAC-TG-3' and JP-2,5'-CAG-GAT-AGA-TIG-GTT-TA-3') complementary to regions of the CBA that flanked the repeats B1, B2, and B3 were constructed (Oligo's Etc.). Genomic DNA from 6 different Staphyloccus aureus strains that were known to bind ¹²⁵I-collagen (Table 1) were isolated as previously described by Lindberg. Polymerase chain reaction (PCR) was performed with a Cetus/Perkin-Elmer DNA Thermocyler. Reaction mixtures (100µl) contained 1mm of each primer, 200 mM of each dNTP, 1 mM Tris-HCl (pH 8.3), 5 mM KC1, 15 mM Mgcl₂, 0.001% gelatin, 3 µg template DNA, and 2.5 U AmpliTaq DNA polymerase. The reaction mixtures were overlayed with 100 µl of mineral oil and amplified for 30 cycles consisting of a 2 minute denaturation at 94°C, a 2 minute annealing period at 55°C, and a 3 minute extension period at 72°C. After amplification, 15 µl of the PCR products were analyzed on a 1% agarose gel (SeaKem GTG, FMC Inc., Rockland, Maine).

PCR analysis of the genomic DNA from the different S. aureus isolates revealed two distinctly different sized products. FDA 574, Cowan, and # 13 all had gene products of 1677 bp, whereas Phillips, #7, and #14391 had gene products of 1118 bp. S. aureus Newman, a known non-collagen binder had no detectable PCR product. There is a direct correlation between the repeat size and the estimated molecular weight of the purified native collagen receptor from the different S. aureus strains tested. Upon further sequence analysis, it appears that a PCR product of 1677 bp corresponds to 3 repeat units, each 560 bp long. A PCR product of 1118 bp therefore corresponds to 2 repeats, each 560 bp long. These data correlate highly with the estimated molecular weight of purified native collagen receptors of 135 kd and 115 kd respectively.

Additional PCR analysis was carried out using primers JP-3(5'-ATA-TGA-ATT-CGA-GTA-TAA-GGA-GGG-GTT-3') and JP-4(5'-ATT-CTG-CAG-AGA-ACT-AAG-AAT-AGC-CTT-3'). These primers flank the intact CBP-gene at the 5' and 3' ends respectively. Using similar PCR parameters, the intact gene could be successfully isolated from S. aureus genomic DNA. Once again two distinctly different size gene products were discovered. Interestingly, the S. aureus isolates which had 3 repeats had a CBP-gene corresponding to 3.5 kb. The S. aureus strains which had only two repeats, had a CBP-gene of 3.0 kb. This work provides direct evidence that the size of the CBP-gene from various S. aureus isolates is directly proportional to the number of repeating units.

Expression of intact CBP-gene. The 3.5 kb PCR product which encompasses the intact gene (primers JP-3, JP-4) was cloned into the prokaryotic expression vector pKK223-3, Pharmacia-LKB. This vector contains an IPTG inducible tac promoter which drives expression of the cloned gene. Upon induction, coomassie staining of a 5-15% SDS-PAGE gel reveals a 135 kd protein. This matches the expected molecular weight of the native collagen receptor 4. This protein will be confirmed soon by western blot and a functional biological assay.

### Immunological relationship of the collagen adhesin from different clinical isolates.

Previous results indicated that antibodies raised against whole cells of the collagen adhesin positive (CA+) strain S. aureus Cowan and its purified collagen adhesin effectively inhibited binding of ¹²⁵I-labeled collagen to the homologous strain (Switalski et al., 1989). These antibodies also effectively inhibited binding of ¹²⁵-I-collagen to all strains binding collagen, which indicates an immunological cross-reactivity of the collagen binding site. To examine the cell surface proteins recognized by these antibodies, they were used to probe Western blots of lysostaphin lysates pri ared from different S. aureus isolates (Figure 3). Lysostaphin digestion releases from the cell surface of S. aureus a number of proteins, around 30 bands can be visualized in the lysates by Coomassie Brilliant Blue staining of the gel (Switalski et al., 1989). The anti-adhesin antibodies recognized a component of Mᵣ 135 kd in the lysate of strain Cowan (Figure 3, lane a), which is in agreement with our previous observations (Switalski et al., 1989). The major immunoreactive protein detected in the lysates of the other collagen adhesin positive strains (CA+) varied in molecular weight and was present as either 110 kd or 135 kd (Figure 3, lanes b through h). No correlation was observed between the apparent size of the immunoreactive protein and the collagen binding capacity of a strain or its origin (bone, synovial fluid). None of the nine non-binding collagen S. aureus strains tested expressed an immunoreactive protein (Figure 3, lane i).

### Collagen adhesin mediated attachment of staphylococci to collagenous substrata.

The relationship between the ability to express a collagen adhesin and the observed localization of an infection within collagen rich tissues prompted us to analyze the role of the cell surface adhesin in bacterial attachment to collagen containing substrates. We initially studied attachment of bacteria to surfaces coated with type II collagen. Results indicated that a collagen coated surface was an excellent attachment substrate for strains which express a surface localized collagen adhesin. The attachment is time dependent and saturable reaching an equilibrium after 3 hours of incubation (Figure 4A). The number of attaching bacteria is not influenced by the size of the adhesin since strains #14 and Phillips, which either express a 135 kd or 110 kd adhesin respectively, attached in equal numbers to the collagen coated substrate. When bacteria were preincubated with anti-adhesin antibodies, against the collagen adhesin from S. aureus strain Cowan, attachment was inhibited in a concentration dependent manner (Figure 4B). This confirmed previous observations on the immunological cross reactivity of the collagen binding site within the collagen adhesin. Attachment of the adhesin negative strains (CA-) was not affected by preincubation with the anti-adhesin antibodies.

### Attachment of S. aureus to cartilage.

Subsequently, we studied the attachment of bacteria to cartilage, in a model mimicking the initial events in the development of infectious arthritis. In this model uniform pieces of cartilage were incubated with ¹²⁵I-surfaced labeled S. aureus. Bovine nasal cartilage which is histologically identical to the bone cartilage was used in this study. Data obtained with cartilage tissue closely resembled those results of collagen coated surfaces. Only CA+ strains attached to cartilage (Figure 4D), exhibiting kinetics analogous to those seen in CA+ strains attaching to collagen coated substrates. This attachment could be completely inhibited by pre-incubation with the anti-adhesin antibodies (Figure 4E). These data indicate, that recognition of tissue collagen maybe sufficient for bacteria to colonize cartilage. Electron microscopy confirmed the quantitative observations presented previously. S. aureus strains which bind ¹²⁵I-collagen and possess an immunoreactive protein on a Western blot, attached in large numbers to cartilage tissue, and can be seen preferentially attaching to collagen fibers (Figure 4C). The number of attaching bacteria is drastically reduced in the presence of anti-adhesin antibodies (Figure 4F). Electron microscopy observations indicated that attachment of bacteria to the bone tissue was indeed related to the ability to express a biologically functional collagen adhesin.

### Creation of artificial bacteria

"Artificial bacteria" were prepared by covalently coating polystyrene beads (1.2 µm vs. staphylococci 0.8 - 1.0 µm in diameter) with the collagen adhesin protein. These beads were then tested in a series of experiments analogous to those performed with intact bacteria. The collagen adhesin (CA) coated beads, but not beads coated with a recombinant form of another staphylococcal cell surface component, the fibronectin receptor (Flock et al., 1987), bound ¹²⁵I-collagen (Figure 5A) in a manner similar to that of CA+ strains of S. aureus (Speziale et al., 1986). This binding was abolished by anti-CA antibodies, whereas preimmune antibodies did not effectively inhibit binding (Figure 5B). When "artificial bacteria" were assayed for the ability to attach to collagen (data not shown) or cartilage, we found that CA beads adhered to the substrate in a time dependent manner, identical to that of CA+ strains of S. aureus, while beads coated with the fibronectin receptor did not adhere at significant levels (Figure 5C). The anti-CA antibody inhibited the adhesion of CA beads to cartilage in a dose dependent fashion, whereas a preimmune antibodies had no effect (Figure 5D). Once again the quantitative binding data was corroborated by electron microscopy observations. CA coated beads attached in large numbers to cartilage tissue, in particular to collagen fibers (Figure 5E), while beads coated with the fibronectin receptor did not (Figure 5F).

### Localization of the collagen binding domain within the collagen adhesin.

Various expression constructs have been created in E. coli in effort to specifically localize the collagen binding domain. Two different types of expression vectors have been utilized in these experiments, pKK223-3 and pGEX-2T, the second of which results in the collagen adhesin fused to glutathione-S-transferase. To date the smallest region of the adhesin which has demonstratable collagen binding activity is contained within construct pGEx-1.1. This fusion protein is approximately 68 kDa, 41 kDa of which is represented by the collagen adhesin. As shown in Figure 6, the collagen binding activity is located within the A domain of the cna gene.

The present collagen binding protein can be used for immunization, whereby the protein, preferably in combination with a fusion protein to create a large antigen to respond to, is injected in dosages causing immunological reaction in the host mammal. Thus the collagen binding protein can be used in vaccination of ruminants against mastitis caused by Staphylococcal infections.

Further, the collagen binding protein can be used to block an infection in an open skin wound by wound treatment using the collagen binding protein in a suspension. Thus the collagen binding protein can be used for the treat-35 ment of wounds, e.g. for blocking protein receptors, or for immunization (vaccination). In the latter case the host body produces specific antibodies, which can protect against invasion of bacterial strains comprising such a collagen binding protein. Hereby the antibodies block the adherence of the bacterial strains to damaged tissue. Treatment of septic arthritis is included as well.

Examples of colonizing of a tissue damage are: a) colonizing of wounds in skin and connective tissue, which wounds have been caused by a mechanical trauma, chemical damage, and/or thermical damage; b) colonizing of wounds on mucous membranes, such as in the mouth cavity, or in the mammary glands, urethra, or vagina; c) colonizing on connective tissue proteins, which have been exposed by a minimal tissue damage (microlesion) in connection with epithelium and endothelium (mastitis, heart valve infection, hip exchange surgery).

When using the present CBP, or the polypeptide, for the purpose of immunization (vaccination) in mammals, including man, the protein, or polypeptide is dispersed in sterile, isotonic saline solution, optionally while adding a pharmaceutically acceptable dispersing agent. Different types of adjuvants can further be used in order to sustain the release in the tissue, and thus expose the protein or the peptide for a longer time to the immundefense system of a body.

A suitable dosage to obtain immunization is 0,5 to 5 µg of CBP, or polypeptide, per kg bodyweight and injection of immunization. In order to obtain a durable immunization, vaccination should be carried out at more than one consecutive occasion with an interval of 1 to 3 weeks, preferably at three occasions.

When using the present CBP, or polypeptide, for topical, local administration the protein is dispersed in an isotonic saline solution to a concentration of 25 to 250 µg per ml. The wounds are then treated with such an amount only to obtain a complete wetting of the wound surface. For an average wound thus only a couple of millilitres of solution are used in this way. After treatment using the protein solution the wounds are suitably washed with isotonic saline or another suitable wound treatment solution.

Further the collagen binding protein as well as the minimal collagen binding site polypeptide, of the present invention can be used to diagnose bacterial infections caused by Staphylococci strains, whereby a collagen binding protein of the present invention is immobilized on a solid carrier, such as small latex or Sepharose^{R} beads, whereupon sera containing antibodies are allowed to pass and react with the CBP thus immobilized. The agglutination is then measured by known methods.

Further, the CBP, or the polypeptide can be used in an ELISA test (Enzyme Linked Immuno Sorbent Assay; E Engvall, Med. Biol. 55, 193, (1977). Hereby wells in a polystyrene microtitre plate are coated with the CBP, and incubated oder night at 4°C. The planes are then thoroughly washed using PBS containing 0,05 % TWEEN 20, and dried. Serial dilution of the patient serum were made in PBS-Tween, were added to the wells, and incubated at 30°C for 1,5 hrs. After rinsing antihuman-IgG conjugated with an enzyme, or an antibovine-IgG conjugated with an enzyme, respectively, horseradishperoxidase or an alkaline phosphatasae, was added to the wells and incubated at 30°C for 1,5 hrs, whereupon when the IgG has been bound thereto, and after rinsing, an enzyme substrate is added, a p-nitrophosphate in case of an alkaline phosphatase, or ortophenylene diamine substrate (OPD) in case a peroxidase has been used, respectively. The plates comprising the wells were thus then rinsed using a citrate buffer containing 0,055 % OPD, and 0,005 % H₂O₂, and incubated at 30°C for 10 min. Enzyme reaction was stopped by adding a 4N solution of H₂SO₄ to each well. The colour development was measured using a spectrophotometer.

Depending on the type of enzyme substrate used a fluoroscense measurement can be used as well.

Another method to diagnose Staphylococcal infections is by using the DNA gene probe method based on the CBP nucleotide sequence or the polypeptide sequence. In the case of diagnozing a mastitis a milk sample is run through a membrane which collects bacteria present. Autolysis of the bacteria in alkali the released single stranded DNA binds to the membrane. The DNA gene probe, optionally labelled enzymatically, or by a radioactive isotope is then added to the membrane comprising the DNA sequence, whereby the DNA 10 gene probe attaches to the sequence where appearing. The enzyme or the radioactive isotope can then readily be determined by known methods.

Above the term collagen binding protein includes the polypeptide sequence as well, which polypeptide sequence forms the minimal collagen binding site of the complete protein.

### References:

Carret, G., H. Emonard, G. Fardel, M. Druguet, D. Herbage, and J. P. Flandrois. 1985. Ann. Inst Pasteur (Paris) 136A:241-245.
Guss, B., M. Uhle'n, B. Nilsson, M. Lindberg, J. Sjöquist and J. Sjödahl. 1981. J. Biochem., 138, 413-420.
Guss, B., M. Eliasson, A. Olsson, M. Uhle'n, A.-K. Frej, H. Jörnvall, J.-I. Flock and M. Lindberg. 1986. EMBO J., 5, 1567-1575.
Holderbaum, D., R.A. Spech and L. A. Ehrhart. 1985. Collagen Relat. Res. 5:261-271.
Holderbaum, D., G. S. Hall and L. A. Ehrhart. 1986. Infect. Immun. 54:359-364.
Hollingshead, S. K., V. A. Fischetti and J. R. Scott. 1986. J. Biol. Chem. 261:1677-1686.
Signs, S., G. Raucci, . Jönsson, P.-E. Lindgren, G. M. Anantharamaiah, M. Höök and M. Lindberg. 1989. Proc. Nutl. Acad. Sci. USA. 86:699-703.
Speziale, P. G. Raucci, L. Visal, L. M. Switalski, R. Timpl and M. Höök. 1986. J. Bact. 167:77-81.
Switalski, L. M., P. Speziale and M. Hook. 1989. J. Biol. Chem. 264:21080-21086.
Uhle'n, M., B. Guss, B. Nilsson, S. Gatenbeck, L. Philipsson and M. Lindberg. 1984. J. Biol. Chem. 259:1695-1702.
Vercellotti, G. M., J. B. McCarthy, . Lindholm, P. K. Peterson, H.S. Jacob and L. T. Furcht. 1985. Am. J. Pathol. 120:13-21.

## Claims

1. A plasmid pSAC104 as contained in the E. coli TG1 having the deposit number DSM 6199.

2. An E. coli strain expressing a collagen binding protein as coded for by the plasmid of claim 1.

3. A micro-organism transformed by the recombinant DNA molecule of claim 1.

4. Hybrid-DNA-molecule according to claim 1, characterized in that it comprises the following nucleotide sequence:

GCA
CGAGATATTT CATCAACGAA TGTTACAGAT TTAACTGTAT CACCGTCTAA
GATAGAAGAT GGTGGTAAAA CGACAGTAAA AATGACGTTC GACGATAAAA
ATGGAAAAAT ACAAAATGGT GACATGATTA AAGTGGCATG GCCGACAAGC
GGTACAGTAA AGATAGAGGG TTATAGTAAA ACAGTACCAT TAACTGTTAA
AGGTGAACAG GTGGGTCAAG CAGTTATTAC ACCAGACGGT GCAACAATTA
CATTCAATGA TAAAGTAGAA AAATTAAGTG ATGTTTCGGG ATTTGCAGAA
TTTGAAGTAC AAGGAAGAAA TTTAACGCAA ACAAATACTT TAGATGACAA
AGTAGCTACG ATAACATCTG GGAATAAATC AACGAATGTT ATCGGTTGGA
TAAAAGTGAA GCGGGAACCA GTAGTGTTTC TAATTAATAA AAGCGGGAAG
ATATGCTACC AAGAAGATAC GACACATGTA CGATGGTTTT TAAATATTAA
CAATGAAAAA AGTTATGTAT CGAAAGATAT TACTATAAAG GATCAGATTC
AAGGTGGACA GCAGTTAGAT TTAAGCACAT TAAACATTAA TGTGACAGGT
ACACATAGCA ATTATTATAG TGGACAAAGT GCAATTACTG ATTTTGAAAA
AGCCTTTCCA GGTTCTAAAA TAACTGTTGA TAATACGAAG AACACAATTG
ATGTAACAAT TCCACAAGGC TATGGGTCAT ATAATAGTTT TTCAATTAAC

TACAAAACCA AAATTACGAA TGAACAGCAA AAAGAGTTTG TTAATAATTC
ACAAGCTTGG TATCAAGAGC ATGGTAAGGA AGAAGTGAAC GGGAAATCAT
TTAATCATAC TGTGCACAAT ATTAATGCTA ATGCCGGTAT TGAAGGTACT
GTAAAAGGTG AATTAAAAGT TTTAAAACAG GATAAAGATA CCAAGGCTCC
TATAGCTAAT GTAAAATTTA AACTTTCTAA AAAAGATGGA TCAGTTGTAA
AGGACAATCA AAAAGAAATT GAGATTATAA CAGATGCAAA CGGTATTGCT
AATATTAAAG CGTTGCCTAG TGGAGACTAT ATTTTAAAAG AAATAGAGGC
GCCACGACCG TATACATTTG ATAAGGATAA AGAATATCCG TTTACTATGA
AAGATACAGA TAATCAGGGA TATTTTACGA CTATTGAAAA TGCAAAAGCG
ATAGAAAAAA CAAAAGATGT TTCTGCTCAA AAGGTTTGGG AAGGCACTCA
AAAAGTGAAA CCAACGATTT ATTTCAAGTT GTACAAACAA GATGACAATC
AAAATACAAC ACCAGTAGAC AAAGCAGAGA TTAAAAAATT AGAAGATGGA
ACGACAAAAG TGACATGGTC TAATCTTCCG GAAAATGACA AAAATGGCAA
GGCTATTAAA TATTTAGTTA AAGAAGTAAA TGCTCAAGGT GAAGATACAA
CACCAGAAGG ATATACTAAA AAAGAAAATG GTTTAGTGGT TACTAATACT
GAAAAACCAA TCGAAACAAC ATCAATTAGT GGTGAAAAAG TATGGGACGA
CAAAGACAAT CAAGATGGTA AGAGACCAGA AAAAGTCAGT GTGAATTTAT
TGGCTAACGG GGAGAAAGTA AAAACGTTAG ACGTGACATC TGAAACAAAC
TGGAAGTACG AATTTAAAGA CTTACCGAAG TATGATGAAG GAAAGAAAAT
AGAATATACA GTGACCGAAG ATCACGTAAA AGACTACACA ACAGACATCA
ACGGTACGAC AATAACGAAC AAGTATACAC CAGGAGAGAC ATCGGCAACA
GTAACAAAAA ATTGGGATGA CAATAATAAC CAAGACGGAA AACGACCAAC
TGAAATCAAA GTTGAGTTAT ATCAAGACGG AAAAGCAACA GGAAAAACGG
CAACATTAAA TGAATCTAAT AACTGGACCC ATACGTGGAC AGGATTAGAT
GAAAAAGCAA AAGGACAACA AGTAAAATAC ACAGTCGAGG AATTAACAAA
GGTCAAAGGT TATACAACAC ATGTGGATAA CAATGATATG GGTAACTTGA
TTGTGACGAA TAAATATACG CCAGAAACAA CATCAATTAG TGGTGAAAAA
GTATGGGACG ACAAAGACAA TCAAGATGGT AAGAGACCAG AAAAAGTCAG
TGTGAATTTA TTGGCTGATG GAGAGAAAGT AAAAACGTTA GACGTGACAT
CTGAAACAAA CTGGAAGTAC GAATTTAAAG ACTTACCGAA GTATGATGAA
GGAAAGAAAA TAGAATATAC AGTGACCGAA GATCACGTAA AAGACTACAC
AACAGACATC AACGGTACGA CAATAACGAA CAAGTATACA CCAGGAGAGA
CATCGGCAAC AGTAACAAAA AATTGGGATG ACAATAATAA CCAAGACGGA
AAACGACCAA CTGAAATCAA AGTTGAGTTA TATCAAGACG GAAAAGCAAC
AGGAAAAACG GCAACATTAA ATGAATCTAA TAACTGGACC CATACGTGGA
CAGGATTAGA TGAAAAAGCA AAAGGACAAC AAGTAAAATA CACAGTCGAG

GAATTAACAA AGGTCAAAGG TTATACAACA CATGTGGATA ACAATGATAT
GGGCAACTTG ATTGTGACGA ATAAATATAC GCCAGAAACA ACATCAATTA
GCGGTGAAAA AGTATGGGAC GACAAAGACA ATCAAGATGG TAAGAGACCA
GAAAAAGTCA GTGTAAATTT ATTGGCTAAC GGAGAGAAAG TAAAAACGTT
AGACGTGACA TCTGAAACAA ACTGGAAGTA CGAATTTAAA GACTTACCGA
AGTATGATGA AGGAAAGAAA ATAGAATATA CAGTGACCGA AGATCACGTA
AAAGACTACA CAACAGACAT CAACGGTACG ACAATAACGA ACAAGTATAC
ACCAGGAGAG ACATCGGCAA CAGTAACAAA AAATTGGGAT GACAATAATA
ACCAAGACGG AAAACGACCA ACTGAAATCA AAGTTGAGTT ATATCAAGAT
GGAAAAGCAA CAGGAAAAAC GGCAATATTA AATGAATCTA ATAACTGGAC
ACATACGTGG ACAGGATTAG ATGAAAAAGC AAAAGGACAA CAAGTAAAAT
ACACAGTCGA TGAATTAACA AAAGTTAATG GCTATACAAC GCATGTGGAT
AACAATGATA TGGGTAACTT GATTGTGACA AATAAATATA CGCCGAAAAA
ACCGAATAAA CCAATCTATC CTGAAAAACC AAAAGACAAA ACACCACCAA
CTAAACCTGA TCATTCTAAT AAAGTTAAAC CAACTCCCCC AGATAAGCCA
TCAAAAGTGG ATAAGGATGA TCAACCTAAA GATAATAAAA CCAAACCTGA
AAATCCTCTA AAAGAATTAC CAAAAACTGG TATGAAGATT ATAACTTCAT
GGATTACATG GGTATTTATA GGTATATTGG GACTGTATTT AATTTTAAGA
AAAAGATTTA ACTCA

5. Plasmid or phage comprising the nucleotide sequence according to claim 4.

6. Micro-organism containing at least a plasmid or phage according to claim 5.

7. A method for producing a collagen binding protein or polypeptide, wherein a) at least one hybrid-DNA-molecule according to claim 4, is introduced into a micro-organism, b) said micro-organism is cultivated in a growth promoting medium, and c) the protein thus formed is isolated by ion exchange chromatography, ammonium sulphate precipitation and gel filtration.

8. Chemical synthesis method for producing a collagen binding protein or polypeptide according to claim 4, whereby an amino acid residue is built up based on said nucleotide sequence encoding for said protein or polypeptide starting from the C-terminal serine, which is stepwise reacted with the appropriate amino acid, whereby it is finally reacted with alanine at the N-terminal end, to form the collagen binding protein.

9. A collagen binding protein comprising the amino acid sequence

Ala
ArgAspIleSerSerThrAsnValThrAspLeuThrValSerProSerLysIleGluAsp
GlyGlyLysThrThrValLysMetThrPheAspAspLysAsnGlyLysIleGlnAsnGly
AspMetIleLysValAlaTrpProThrSerGlyThrValLysIleGluGlyTyrSerLys
ThrValProLeuThrValLysGlyGluGlnValGlyGlnAlaValIleThrProAspGly
AlaThrIleThrPheAsnAspLysValGluLysLeuSerAspValSerGlyPheAlaGlu
PheGluValGlnGlyArgAsnLeuThrGlnThrAsnThrLeuAspAspLysValAlaThr
IleThrSerGlyAsnLysSerThrAsnValIleGlyTrpIleLysValLysArgGluPro
ValValPheLeuIleAsnLysSerGlyLysIleCysTyrGlnGluAspThrThrHisVal
ArgTrpPheLeuAsnIleAsnAsnGluLysSerTyrValSerLysAspIleThrIleLys
AspGlnIleGlnGlyGlyGlnGlnLeuAspLeuSerThrLeuAsnIleAsnValThrGly
ThrHisSerAsnTyrTyrSerGlyGlnSerAlaIleThrAspPheGluLysAlaPhePro
GlySerLysIleThrValAspAsnThrLysAsnThrIleAspValThrIleProGlnGly
TyrGlySerTyrAsnSerPheSerIleAsnTyrLysThrLysIleThrAsnGluGlnGln
LysGluPheValAsnAsnSerGlnAlaTrpTyrGlnGluHisGlyLysGluGluValAsn
GlyLysSerPheAsnHisThrValHisAsnIleAsnAlaAsnAlaGlyIleGluGlyThr
ValLysGlyGluLeuLysValLeuLysGlnAspLysAspThrLysAlaProIleAlaAsn
ValLysPheLysLeuSerLysLysAspGlySerValValLysAspAsnGlnLysGluIle
GluIleIleThrAspAlaAsnGlyIleAlaAsnIleLysAlaLeuProSerGlyAspTyr
IleLeuLysGluIleGluAlaProArgProTyrThrPheAspLysAspLysGluTyrPro
PheThrMetLysAspThrAspAsnGlnGlyTyrPheThrThrIleGluAsnAlaLysAla
IleGluLysThrLysAspValSerAlaGlnLysValTrpGluGlyThrGlnLysValLys
ProThrIleTyrPheLysLeuTyrLysGlnAspAspAsnGlnAsnThrThrProValAsp
LysAlaGluIleLysLysLeuGluAspGlyThrThrLysValThrTrpSerAsnLeuPro
GluAsnAspLysAsnGlyLysAlaIleLysTyrLeuValLysGluValAsnAlaGlnGly

GluAspThrThrProGluGlyTyrThrLysLysGluAsnGlyLeuValValThrAsnThr
GluLysProIleGluThrThrSerIleSerGlyGluLysValTrpAspAspLysAspAsn
GlnAspGlyLysArgProGluLysValSerValAsnLeuLeuAlaAsnGlyGluLysVal
LysThrLeuAspValThrSerGluThrAsnTrpLysTyrGluPheLysAspLeuProLys
TyrAspGluGlyLysLysIleGluTyrThrValThrGluAspHisValLysAspTyrThr
ThrAspIleAsnGlyThrThrIleThrAsnLysTyrThrProGlyGluThrSerAlaThr
ValThrLysAsnTrpAspAspAsnAsnAsnGlnAspGlyLysArgProThrGluIleLys
ValGluLeuTyrGlnAspGlyLysAlaThrGlyLysThrAlaThrLeuAsnGluSerAsn
AsnTrpThrHisThrTrpThrGlyLeuAspGluLysAlaLysGlyGlnGlnValLysTyr
ThrValGluGluLeuThrLysValLysGlyTyrThrThrHisValAspAsnAsnAspMet
GlyAsnLeuIleValThrAsnLysTyrThrProGluThrThrSerIleSerGlyGluLys
ValTrpAspAspLysAspAsnGlnAspGlyLysArgProGluLysValSerValAsnLeu
LeuAlaAspGlyGluLysValLysThrLeuAspValThrSerGluThrAsnTrpLysTyr
GluPheLysAspLeuProLysTyrAspGluGlyLysLysIleGluTyrThrValThrGlu
AspHisValLysAspTyrThrThrAspIleAsnGlyThrThrIleThrAsnLysTyrThr
ProGlyGluThrSerAlaThrValThrLysAsnTrpAspAspAsnAsnAsnGlnAspGly
LysArgProThrGluIleLysValGluLeuTyrGlnAspGlyLysAlaThrGlyLysThr
AlaThrLeuAsnGluSerAsnAsnTrpThrHisThrTrpThrGlyLeuAspGluLysAla
LysGlyGlnGlnValLysTyrThrValGluGluLeuThrLysValLysGlyTyrThrThr
HisValAspAsnAsnAspMetGlyAsnLeuIleValThrAsnLysTyrThrProGluThr
ThrSerIleSerGlyGluLysValTrpAspAspLysAspAsnGlnAspGlyLysArgPro
GluLysValSerValAsnLeuLeuAlaAsnGlyGluLysValLysThrLeuAspValThr
SerGluThrAsnTrpLysTyrGluPheLysAspLeuProLysTyrAspGluGlyLysLys
IleGluTyrThrValThrGluAspHisValLysAspTyrThrThrAspIleAsnGlyThr
ThrIleThrAsnLysTyrThrProGlyGluThrSerAlaThrValThrLysAsnTrpAsp
AspAsnAsnAsnGlnAspGlyLysArgProThrGluIleLysValGluLeuTyrGlnAsp
GlyLysAlaThrGlyLysThrAlaIleLeuAsnGluSerAsnAsnTrpThrHisThrTrp
ThrGlyLeuAspGluLysAlaLysGlyGlnGlnValLysTyrThrValAspGluLeuThr
LysValAsnGlyTyrThrThrHisValAspAsnAsnAspMetGlyAsnLeuIleValThr
AsnLysTyrThrProLysLysProAsnLysProIleTyrProGluLysProLysAspLys
ThrProProThrLysProAspHisSerAsnLysValLysProThrProProAspLysPro
SerLysValAspLysAspAspGlnProLysAspAsnLysThrLysProGluAsnProLeu
LysGluLeuProLysThrGlyMetLysIleIleThrSerTrpIleThrTrpValPheIle
GlyIleLeuGlyLeuTyrLeuIleLeuArgLysArgPheAsnSer

## Patentansprüche

1. Plasmid pSAC104 in E. coli TG1 mit Hinterlegungsnummer DSM 6199.

2. Ein E. coli Stamm der ein Kollagen-bindendes Protein expressioniert welches von das Plasmid nach Anspruch 1 kodiert ist.

3. Mikroorganismus transformiert von das Rekombinant DNA Molekül nach Anspruch 1.

4. Hybrid-DNA Molekül nach Anspruch 1, dadurch gekennzeichnet, daß es die folgende Nukleotidsequenz umfaßt:

GCA
CGAGATATTT CATCAACGAA TGTTACAGAT TTAACTGTAT CACCGTCTAA
GATAGAAGAT GGTGGTAAAA CGACAGTAAA AATGACGTTC GACGATAAAA
ATGGAAAAAT ACAAAATGGT GACATGATTA AAGTGGCATG GCCGACAAGC
GGTACAGTAA AGATAGAGGG TTATAGTAAA ACAGTACCAT TAACTGTTAA
AGGTGAACAG GTGGGTCAAG CAGTTATTAC ACCAGACGGT GCAACAATTA
CATTCAATGA TAAAGTAGAA AAATTAAGTG ATGTTTCGGG ATTTGCAGAA
TTTGAAGTAC AAGGAAGAAA TTTAACGCAA ACAAATACTT TAGATGACAA
AGTAGCTACG ATAACATCTG GGAATAAATC AACGAATGTT ATCGGTTGGA
TAAAAGTGAA GCGGGAACCA GTAGTGTTTC TAATTAATAA AAGCGGAAG
ATATGCTACC AAGAAGATAC GACACATGTA CGATGGTTTT TAAATATTAA
CAATGAAAAA AGTTATGTAT CGAAAGATAT TACTATAAAG GATCAGATTC
AAGGTGGACA GCAGTTAGAT TTAAGCACAT TAAACATTAA TGTGACAGGT
ACACATAGCA ATTATTATAG TGGACAAAGT GCAATTACTG ATTTTGAAAA
AGCCTTTCCA GGTTCTAAAA TAACTGTTGA TAATACGAAG AACACAATTG
ATGTAACAAT TCCACAAGGC TATGGGTCAT ATAATAGTTT TTCAATTAAC

TACAAAACCA AAATTACGAA TGAACAGCAA AAAGAGTTTG TTAATAATTC
ACAAGCTTGG TATCAAGAGC ATGGTAAGGA AGAAGTGAAC GGGAAATCAT
TTAATCATAC TGTGCACAAT ATTAATGCTA ATGCCGGTAT TGAAGGTACT
GTAAAAGGTG AATTAAAAGT TTTAAAACAG GATAAAGATA CCAAGGCTCC
TATAGCTAAT GTAAAATTTA AACTTTCTAA AAAAGATGGA TCAGTTGTAA
AGGACAATCA AAAAGAAATT GAGATTATAA CAGATGCAAA CGGTATTGCT
AATATTAAAG CGTTGCCTAG TGGAGACTAT ATTTTAAAAG AAATAGAGGC
GCCACGACCG TATACATTTG ATAAGGATAA AGAATATCCG TTTACTATGA
AAGATACAGA TAATCAGGGA TATTTTACGA CTATTGAAAA TGCAAAAGCG
ATAGAAAAAA CAAAAGATGT TTCTGCTCAA AAGGTTTGGG AAGGCACTCA
AAAAGTGAAA CCAACGATTT ATTTCAAGTT GTACAAACAA GATGACAATC
AAAATACAAC ACCAGTAGAC AAAGCAGAGA TTAAAAAATT AGAAGATGGA
ACGACAAAAG TGACATGGTC TAATCTTCCG GAAAATGACA AAAATGGCAA
GGCTATTAAA TATTTAGTTA AAGAAGTAAA TGCTCAAGGT GAAGATACAA
CACCAGAAGG ATATACTAAA AAAGAAAATG GTTTAGTGGT TACTAATACT
GAAAAACCAA TCGAAACAAC ATCAATTAGT GGTGAAAAAG TATGGGACGA
CAAAGACAAT CAAGATGGTA AGAGACCAGA AAAAGTCAGT GTGAATTTAT
TGGCTAACGG GGAGAAAGTA AAAACGTTAG ACGTGACATC TGAAACAAAC
TGGAAGTACG AATTTAAAGA CTTACCGAAG TATGATGAAG GAAAGAAAAT
AGAATATACA GTGACCGAAG ATCACGTAAA AGACTACACA ACAGACATCA
ACGGTACGAC AATAACGAAC AAGTATACAC CAGGAGAGAC ATCGGCAACA
GTAACAAAAA ATTGGGATGA CAATAATAAC CAAGACGGAA AACGACCAAC
TGAAATCAAA GTTGAGTTAT ATCAAGACGG AAAAGCAACA GGAAAAACGG
CAACATTAAA TGAATCTAAT AACTGGACCC ATACGTGGAC AGGATTAGAT
GAAAAAGCAA AAGGACAACA AGTAAAATAC ACAGTCGAGG AATTAACAAA
GGTCAAAGGT TATACAACAC ATGTGGATAA CAATGATATG GGTAACTTGA
TTGTGACGAA TAAATATACG CCAGAAACAA CATCAATTAG TGGTGAAAAA
GTATGGGACG ACAAAGACAA TCAAGATGGT AAGAGACCAG AAAAAGTCAG
TGTGAATTTA TTGGCTGATG GAGAGAAAGT AAAAACGTTA GACGTGACAT
CTGAAACAAA CTGGAAGTAC GAATTTAAAG ACTTACCGAA GTATGATGAA
GGAAAGAAAA TAGAATATAC AGTGACCGAA GATCACGTAA AAGACTACAC
AACAGACATC AACGGTACGA CAATAACGAA CAAGTATACA CCAGGAGAGA
CATCGGCAAC AGTAACAAAA AATTGGGATG ACAATAATAA CCAAGACGGA
AAACGACCAA CTGAAATCAA AGTTGAGTTA TATCAAGACG GAAAAGCAAC
AGGAAAAACG GCAACATTAA ATGAATCTAA TAACTGGACC CATACGTGGA
CAGGATTAGA TGAAAAAGCA AAAGGACAAC AAGTAAAATA CACAGTCGAG

GAATTAACAA AGGTCAAAGG TTATACAACA CATGTGGATA ACAATGATAT
GGGCAACTTG ATTGTGACGA ATAAATATAC GCCAGAAACA ACATCAATTA
GCGGTGAAAA AGTATGGGAC GACAAAGACA ATCAAGATGG TAAGAGACCA
GAAAAAGTCA GTGTAAATTT ATTGGCTAAC GGAGAGAAAG TAAAAACGTT
AGACGTGACA TCTGAAACAA ACTGGAAGTA CGAATTTAAA GACTTACCGA
AGTATGATGA AGGAAAGAAA ATAGAATATA CAGTGACCGA AGATCACGTA
AAAGACTACA CAACAGACAT CAACGGTACG ACAATAACGA ACAAGTATAC
ACCAGGAGAG ACATCGGCAA CAGTAACAAA AAATTGGGAT GACAATAATA
ACCAAGACGG AAAACGACCA ACTGAAATCA AAGTTGAGTT ATATCAAGAT
GGAAAAGCAA CAGGAAAAAC GGCAATATTA AATGAATCTA ATAACTGGAC
ACATACGTGG ACAGGATTAG ATGAAAAAGC AAAAGGACAA CAAGTAAAAT
ACACAGTCGA TGAATTAACA AAAGTTAATG GCTATACAAC GCATGTGGAT
AACAATGATA TGGGTAACTT GATTGTGACA AATAAATATA CGCCGAAAAA
ACCGAATAAA CCAATCTATC CTGAAAAACC AAAAGACAAA ACACCACCAA
CTAAACCTGA TCATTCTAAT AAAGTTAAAC CAACTCCCCC AGATAAGCCA
TCAAAAGTGG ATAAGGATGA TCAACCTAAA GATAATAAAA CCAAACCTGA
AAATCCTCTA AAAGAATTAC CAAAAACTGG TATGAAGATT ATAACTTCAT
GGATTACATG GGTATTTATA GGTATATTGG GACTGTATTT AATTTTAAGA
AAAAGATTTA ACTCA

5. Plasmid oder Phage mit der Nukleotidsequenz nach Anspruch 4.

6. Mikroorganismus mit mindestens einem Plasmid oder Phagen nach Anspruch 5.

7. Verfahren für Produktion eines Kollagen-bindendes Protein oder Polypeptid, wobei a) mindenstens ein Hybrid-DNA Molekül nach Anspruch lin einem Mikroorganismus eingeführt wird, b) besagte Mikroorganismus in einem Wachstumsmedium kultiviert wird, und c) das Protein so enthaltend bei lonenaustauscherchromatographie, Ammoniumsulphatefällung und Gelfiltrierung isoliert wird.

8. Chemische Synthese des Kollagen-bindendes Protein oder Polypeptid nach Anspruch 4, wobei eine Aminosäuresequenz aufgebaut wird, basierend auf besagter Nukleotidsequenz , die für besagtes Protein kodiert, beginnend beim C-terminalen Serin, welches stufenweise mit den entsprechenden Aminosäure umgesetzt wird, wobei es schließlich am N-terminalen Ende mit Alanin verbunden wird um den Kollagen-bindendes Protein zu bilden.

9. Kollagen-bindendes Protein mit der Aminosäuresequenz

Ala
ArgAspIleSerSerThrAsnValThrAspLeuThrValSerProSerLysIleGluAsp
GlyGlyLysThrThrValLysMetThrPheAspAspLysAsnGlyLysIleGlnAsnGly
AspMetIleLysValAlaTrpProThrSerGlyThrValLysIleGluGlyTyrSerLys
ThrValProLeuThrValLysGlyGluGlnValGlyGlnAlaValIleThrProAspGly
AlaThrIleThrPheAsnAspLysValGluLysLeuSerAspValSerGlyPheAlaGlu
PheGluValGlnGlyArgAsnLeuThrGlnThrAsnThrLeuAspAspLysValAlaThr
IleThrSerGlyAsnLysSerThrAsnValIleGlyTrpIleLysValLysArgGluPro
ValValPheLeuIleAsnLysSerGlyLysIleCysTyrGlnGluAspThrThrHisVal
ArgTrpPheLeuAsnIleAsnAsnGluLysSerTyrValSerLysAspIleThrIleLys
AspGlnIleGlnGlyGlyGlnGlnLeuAspLeuSerThrLeuAsnIleAsnValThrGly
ThrHisSerAsnTyrTyrSerGlyGlnSerAlaIleThrAspPheGluLysAlaPhePro
GlySerLysIleThrValAspAsnThrLysAsnThrIleAspValThrIleProGlnGly
TyrGlySerTyrAsnSerPheSerIleAsnTyrLysThrLysIleThrAsnGluGlnGln
LysGluPheValAsnAsnSerGlnAlaTrpTyrGlnGluHisGlyLysGluGluValAsn
GlyLysSerPheAsnHisThrValHisAsnIleAsnAlaAsnAlaGlyIleGluGlyThr
ValLysGlyGluLeuLysValLeuLysGlnAspLysAspThrLysAlaProIleAlaAsn
ValLysPheLysLeuSerLysLysAspGlySerValValLysAspAsnGlnLysGluIle
GluIleIleThrAspAlaAsnGlyIleAlaAsnIleLysAlaLeuProSerGlyAspTyr
IleLeuLysGluIleGluAlaProArgProTyrThrPheAspLysAspLysGluTyrPro
PheThrMetLysAspThrAspAsnGlnGlyTyrPheThrThrIleGluAsnAlaLysAla
IleGluLysThrLysAspValSerAlaGlnLysValTrpGluGlyThrGlnLysValLys
ProThrIleTyrPheLysLeuTyrLysGlnAspAspAsnGlnAsnThrThrProValAsp
LysAlaGluIleLysLysLeuGluAspGlyThrThrLysValThrTrpSerAsnLeuPro
GluAsnAspLysAsnGlyLysAlaIleLysTyrLeuValLysGluValAsnAlaGlnGly

GluAspThrThrProGluGlyTyrThrLysLysGluAsnGlyLeuValValThrAsnThr
GluLysProIleGluThrThrSerIleSerGlyGluLysValTrpAspAspLysAspAsn
GlnAspGlyLysArgProGluLysValSerValAsnLeuLeuAlaAsnGlyGluLysVal
LysThrLeuAspValThrSerGluThrAsnTrpLysTyrGluPheLysAspLeuProLys
TyrAspGluGlyLysLysIleGluTyrThrValThrGluAspHisValLysAspTyrThr
ThrAspIleAsnGlyThrThrIleThrAsnLysTyrThrProGlyGluThrSerAlaThr
ValThrLysAsnTrpAspAspAsnAsnAsnGlnAspGlyLysArgProThrGluIleLys
ValGluLeuTyrGlnAspGlyLysAlaThrGlyLysThrAlaThrLeuAsnGluSerAsn
AsnTrpThrHisThrTrpThrGlyLeuAspGluLysAlaLysGlyGlnGlnValLysTyr
ThrValGluGluLeuThrLysValLysGlyTyrThrThrHisValAspAsnAsnAspMet
GlyAsnLeuIleValThrAsnLysTyrThrProGluThrThrSerIleSerGlyGluLys
ValTrpAspAspLysAspAsnGlnAspGlyLysArgProGluLysValSerValAsnLeu
LeuAlaAspGlyGluLysValLysThrLeuAspValThrSerGluThrAsnTrpLysTyr
GluPheLysAspLeuProLysTyrAspGluGlyLysLysIleGluTyrThrValThrGlu
AspHisValLysAspTyrThrThrAspIleAsnGlyThrThrIleThrAsnLysTyrThr
ProGlyGluThrSerAlaThrValThrLysAsnTrpAspAspAsnAsnAsnGlnAspGly
LysArgProThrGluIleLysValGluLeuTyrGlnAspGlyLysAlaThrGlyLysThr
AlaThrLeuAsnGluSerAsnAsnTrpThrHisThrTrpThrGlyLeuAspGluLysAla
LysGlyGlnGlnValLysTyrThrValGluGluLeuThrLysValLysGlyTyrThrThr
HisValAspAsnAsnAspMetGlyAsnLeuIleValThrAsnLysTyrThrProGluThr
ThrSerIleSerGlyGluLysValTrpAspAspLysAspAsnGlnAspGlyLysArgPro
GluLysValSerValAsnLeuLeuAlaAsnGlyGluLysValLysThrLeuAspValThr
SerGluThrAsnTrpLysTyrGluPheLysAspLeuProLysTyrAspGluGlyLysLys
IleGluTyrThrValThrGluAspHisValLysAspTyrThrThrAspIleAsnGlyThr
ThrIleThrAsnLysTyrThrProGlyGluThrSerAlaThrValThrLysAsnTrpAsp
AspAsnAsnAsnGlnAspGlyLysArgProThrGluIleLysValGluLeuTyrGlnAsp
GlyLysAlaThrGlyLysThrAlaIleLeuAsnGluSerAsnAsnTrpThrHisThrTrp
ThrGlyLeuAspGluLysAlaLysGlyGlnGlnValLysTyrThrValAspGluLeuThr
LysValAsnGlyTyrThrThrHisValAspAsnAsnAspMetGlyAsnLeuIleValThr
AsnLysTyrThrProLysLysProAsnLysProIleTyrProGluLysProLysAspLys
ThrProProThrLysProAspHisSerAsnLysValLysProThrProProAspLysPro
SerLysValAspLysAspAspGlnProLysAspAsnLysThrLysProGluAsnProLeu
LysGluLeuProLysThrGlyMetLysIleIleThrSerTrpIleThrTrpValPheIle
GlyIleLeuGlyLeuTyrLeuIleLeuArgLysArgPheAsnSer

## Revendications

1. Plasmide pSAC104 contenant dans E. coli TG1 avec le numero de depot DSM 6199.

2. Un E. coli souche expressant une protéine de liasion du collagene, la plasmide de la revendication 1 codant pour cette protéine.

3. Micro-organisme transformée par la molécule d'DNA recombinant de la revendication 1.

4. Molécule d'DNA hybride de la revendication 1, caractérisée en ce quelle comprend la sequence nucléotidique suivante:

GCA
CGAGATATTT CATCAACGAA TGTTACAGAT TTAACTGTAT CACCGTCTAA
GATAGAAGAT GGTGGTAAAA CGACAGTAAA AATGACGTTC GACGATAAAA
ATGGAAAAAT ACAAAATGGT GACATGATTA AAGTGGCATG GCCGACAAGC
GGTACAGTAA AGATAGAGGG TTATAGTAAA ACAGTACCAT TAACTGTTAA
AGGTGAACAG GTGGGTCAAG CAGTTATTAC ACCAGACGGT GCAACAATTA
CATTCAATGA TAAAGTAGAA AAATTAAGTG ATGTTTCGGG ATTTGCAGAA
TTTGAAGTAC AAGGAAGAAA TTTAACGCAA ACAAATACTT TAGATGACAA
AGTAGCTACG ATAACATCTG GGAATAAATC AACGAATGTT ATCGGTTGGA
TAAAAGTGAA GCGGGAACCA GTAGTGTTTC TAATTAATAA AAGCGGGAAG
ATATGCTACC AAGAAGATAC GACACATGTA CGATGGTTTT TAAATATTAA
CAATGAAAAA AGTTATGTAT CGAAAGATAT TACTATAAAG GATCAGATTC
AAGGTGGACA GCAGTTAGAT TTAAGCACAT TAAACATTAA TGTGACAGGT
ACACATAGCA ATTATTATAG TGGACAAAGT GCAATTACTG ATTTTGAAAA
AGCCTTTCCA GGTTCTAAAA TAACTGTTGA TAATACGAAG AACACAATTG
ATGTAACAAT TCCACAAGGC TATGGGTCAT ATAATAGTTT TTCAATTAAC

TACAAAACCA AAATTACGAA TGAACAGCAA AAAGAGTTTG TTAATAATTC
ACAAGCTTGG TATCAAGAGC ATGGTAAGGA AGAAGTGAAC GGGAAATCAT
TTAATCATAC TGTGCACAAT ATTAATGCTA ATGCCGGTAT TGAAGGTACT
GTAAAAGGTG AATTAAAAGT TTTAAAACAG GATAAAGATA CCAAGGCTCC
TATAGCTAAT GTAAAATTTA AACTTTCTAA AAAAGATGGA TCAGTTGTAA
AGGACAATCA AAAAGAAATT GAGATTATAA CAGATGCAAA CGGTATTGCT
AATATTAAAG CGTTGCCTAG TGGAGACTAT ATTTTAAAAG AAATAGAGGC
GCCACGACCG TATACATTTG ATAAGGATAA AGAATATCCG TTTACTATGA
AAGATACAGA TAATCAGGGA TATTTTACGA CTATTGAAAA TGCAAAAGCG
ATAGAAAAAA CAAAAGATGT TTCTGCTCAA AAGGTTTGGG AAGGCACTCA
AAAAGTGAAA CCAACGATTT ATTTCAAGTT GTACAAACAA GATGACAATC
AAAATACAAC ACCAGTAGAC AAAGCAGAGA TTAAAAAATT AGAAGATGGA
ACGACAAAAG TGACATGGTC TAATCTTCCG GAAAATGACA AAAATGGCAA
GGCTATTAAA TATTTAGTTA AAGAAGTAAA TGCTCAAGGT GAAGATACAA
CACCAGAAGG ATATACTAAA AAAGAAAATG GTTTAGTGGT TACTAATACT
GAAAAACCAA TCGAAACAAC ATCAATTAGT GGTGAAAAAG TATGGGACGA
CAAAGACAAT CAAGATGGTA AGAGACCAGA AAAAGTCAGT GTGAATTTAT
TGGCTAACGG GGAGAAAGTA AAAACGTTAG ACGTGACATC TGAAACAAAC
TGGAAGTACG AATTTAAAGA CTTACCGAAG TATGATGAAG GAAAGAAAAT
AGAATATACA GTGACCGAAG ATCACGTAAA AGACTACACA ACAGACATCA
ACGGTACGAC AATAACGAAC AAGTATACAC CAGGAGAGAC ATCGGCAACA
GTAACAAAAA ATTGGGATGA CAATAATAAC CAAGACGGAA AACGACCAAC
TGAAATCAAA GTTGAGTTAT ATCAAGACGG AAAAGCAACA GGAAAAACGG
CAACATTAAA TGAATCTAAT AACTGGACCC ATACGTGGAC AGGATTAGAT
GAAAAAGCAA AAGGACAACA AGTAAAATAC ACAGTCGAGG AATTAACAAA
GGTCAAAGGT TATACAACAC ATGTGGATAA CAATGATATG GGTAACTTGA
TTGTGACGAA TAAATATACG CCAGAAACAA CATCAATTAG TGGTGAAAAA
GTATGGGACG ACAAAGACAA TCAAGATGGT AAGAGACCAG AAAAAGTCAG
TGTGAATTTA TTGGCTGATG GAGAGAAAGT AAAAACGTTA GACGTGACAT
CTGAAACAAA CTGGAAGTAC GAATTTAAAG ACTTACCGAA GTATGATGAA
GGAAAGAAAA TAGAATATAC AGTGACCGAA GATCACGTAA AAGACTACAC
AACAGACATC AACGGTACGA CAATAACGAA CAAGTATACA CCAGGAGAGA
CATCGGCAAC AGTAACAAAA AATTGGGATG ACAATAATAA CCAAGACGGA
AAACGACCAA CTGAAATCAA AGTTGAGTTA TATCAAGACG GAAAAGCAAC
AGGAAAAACG GCAACATTAA ATGAATCTAA TAACTGGACC CATACGTGGA
CAGGATTAGA TGAAAAAGCA AAAGGACAAC AAGTAAAATA CACAGTCGAG

GAATTAACAA AGGTCAAAGG TTATACAACA CATGTGGATA ACAATGATAT
GGGCAACTTG ATTGTGACGA ATAAATATAC GCCAGAAACA ACATCAATTA
GCGGTGAAAA AGTATGGGAC GACAAAGACA ATCAAGATGG TAAGAGACCA
GAAAAAGTCA GTGTAAATTT ATTGGCTAAC GGAGAGAAAG TAAAAACGTT
AGACGTGACA TCTGAAACAA ACTGGAAGTA CGAATTTAAA GACTTACCGA
AGTATGATGA AGGAAAGAAA ATAGAATATA CAGTGACCGA AGATCACGTA
AAAGACTACA CAACAGACAT CAACGGTACG ACAATAACGA ACAAGTATAC
ACCAGGAGAG ACATCGGCAA CAGTAACAAA AAATTGGGAT GACAATAATA
ACCAAGACGG AAAACGACCA ACTGAAATCA AAGTTGAGTT ATATCAAGAT
GGAAAAGCAA CAGGAAAAAC GGCAATATTA AATGAATCTA ATAACTGGAC
ACATACGTGG ACAGGATTAG ATGAAAAAGC AAAAGGACAA CAAGTAAAAT
ACACAGTCGA TGAATTAACA AAAGTTAATG GCTATACAAC GCATGTGGAT
AACAATGATA TGGGTAACTT GATTGTGACA AATAAATATA CGCCGAAAAA
ACCGAATAAA CCAATCTATC CTGAAAAACC AAAAGACAAA ACACCACCAA
CTAAACCTGA TCATTCTAAT AAAGTTAAAC CAACTCCCCC AGATAAGCCA
TCAAAAGTGG ATAAGGATGA TCAACCTAAA GATAATAAAA CCAAACCTGA
AAATCCTCTA AAAGAATTAC CAAAAACTGG TATGAAGATT ATAACTTCAT
GGATTACATG GGTATTTATA GGTATATTGG GACTGTATTT AATTTTAAGA
AAAAGATTTA ACTCA

5. Plasmide ou phage comprenant la sequence nucléotidique de la revendication 4.

6. Micro-organisme contenant au moins un plasmide ou phage suivant la revendication 5.

7. Procedé pour la production une protein ou polypeptide de liasion du collagene dans lequel a) au moins une molécule d'DNA hybride de la revendication 4, soit transféréeée dans un micro-organisme, b) culture de ce micro-organisme dans un millieu de croissance et c) d'isolement de la proteine ainsi formée au moyen d'une chromatographie d'echange ionique, precipitation de ammonium sulphatique et filtration de gel.

8. Synthèse chimique pour la production une protéine ou polypeptide de liasion du collagene de la revendication 4, de telle sorte qu'une séquence d'acides aminés soit formée sur la base de la séquence nucléotidique précitée codant pour ladite protéine en partant de la serine C-terminale que l'on fait réagir progressivement avec l'acide aminé approprié, de sorte qu'elle réagit finalement avec de l'alanine à l'extrémité N-terminale, pour former la protein de liasion du collagene.

9. Protéine ou polypeptide liant le collagene comportant la séquence d'acides aminés:
